(19) Europäisches Patentamt
European Patent Office
Office européen des brevets

(11) Numéro de publication : 0 362 309 B1

# (12) FASCICULE DE BREVET EUROPEEN

(45) Date de publication du fascicule du brevet : 23.09.92 Bulletin 92/39

(51) Int. Cl.[5] : C07D 317/72, C07C 59/86

(21) Numéro de dépôt : 89902223.0

(22) Date de dépôt : 24.02.89

(86) Numéro de dépôt international : PCT/CH89/00036

(87) Numéro de publication internationale : WO 89/09215 05.10.89 Gazette 89/24

## (54) (ETHYLENEDIOXO-3,3 CYCLOHEXYL)-4 ACETOPHENONE ET DERIVES DE CE COMPOSE, PROCEDES POUR LEUR PREPARATION ET UTILISATION DE CES COMPOSES.

(30) Priorité : 21.03.88 CH 1045/88

(43) Date de publication de la demande : 11.04.90 Bulletin 90/15

(45) Mention de la délivrance du brevet : 23.09.92 Bulletin 92/39

(84) Etats contractants désignés : AT BE DE GB IT LU NL SE

(56) Documents cités : FR-A- 2 150 788

(73) Titulaire : SOCHINAZ, SOCIETE CHIMMIQUE DE VIONNAZ S.A.
Chimique de Vinnaz S.A., Rue Principale
CH-1891 Vionnaz (CH)

(72) Inventeur : HAIDER, Akhtar
20, rue de la Mouline
CH-1022 Chavannes (CH)

(74) Mandataire : Kovacs, Paul et al
WILLIAM BLANC & CIE 9, rue du Valais
CH-1202 Genève (CH)

EP 0 362 309 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objets l'(éthylène-dioxo-3,3 cyclohexyl)-4 acétophénone et des dérivés de ce composé ainsi que des procédés pour leur préparation et l'utilisation de ces composés comme produit de départ pour la préparation de l'acide [(oxo-3-cyclohexyl)-4 phényl]-2 propionique et de ses dérivés.

L'acide [(oxo-3-cyclohexyl)-4 phényl]-2 propionique et des dérivés de celui-ci sont connus comme agents anti-inflammatoires non stéroidiens (AINS) qui présentent, par rapport à leurs homologues du type aryl-2-propionique, l'avantage d'avoir une plus grande activité et une moindre toxicité. De tels composés et un procédé pour leur préparation sont décrits dans le brevet français No 72 29436.

Les procédés de préparation connus de ces composés comprennent plusieurs étapes de synthèses gui nécessitent la séparation de régioisomères. Ces procédés présentent l'inconvénient d'être difficilement réalisables à l'échelle industrielle et d'avoir des rendements relativement faibles.

L'invention a pour but d'éliminer ces inconvénients et de permettre l'obtention de l'acide [(oxo-3-cyclohexyl)-4 phényl]-2 propionique et de dérivés de celui-ci de manière efficace et rapide avec un rendement élevé.

A cet effet, conformément à l'invention, le procédé de préparation de l'acide [(oxo-3 cyclohexyl)-4 phényl]-2 propionique et de dérivés de celui-ci, de formule

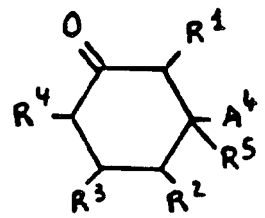

(I)

dans laquelle $A^4$ représente un groupement

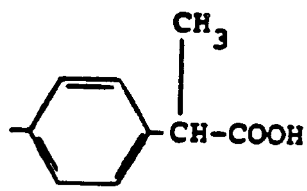

et $R^1$, $R^2$, $R^3$, $R^4$, $R^5$, qui peuvent être identiques ou différents représentent chacun l'hydrogène ou un groupe alkyle, est caractérisé en ce que l'on transforme un composé de formule

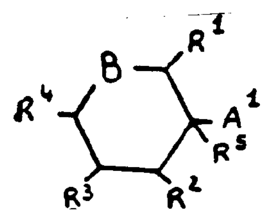

(IV)

dans laquelle $A^1$ représente un groupement

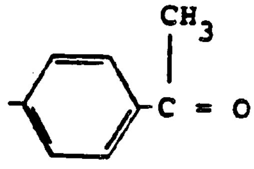

et B représente un groupement

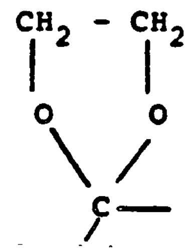

en composé de formule (I).

Conformément à une première forme de mise en oeuvre de ce procédé, on effectue la transformation des composés de formule (IV) en composés de formule (I), en formant des composés intermédiaires successifs de formule:

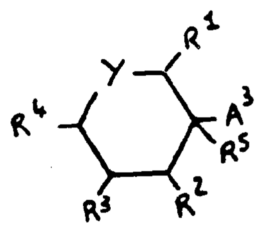

(IX), (X), (XI)
(XII), (XIII), (XIV)

dans lesquelles Y représente, soit le groupement B défini dans la revendication 1, soit un groupement

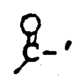

et $A^3$ représente un groupement

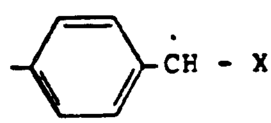

dans lequel X représente OH, Cl ou CN, les significations de Y et X pour chaque formule étant telles qu'indiquées dans le tableau suivant:

| Y = | | B | 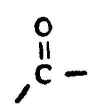 |
|---|---|---|---|
| X = | OH | (IX) | (XII) |
| | Cl | (X) | (XIII) |
| | CN | (XI) | (XIV) |

à savoir, un premier composé intermédiaire (IX) ou (XII), dans lequel X représente OH, obtenu par réduction de la fonction cétone du groupement $A^1$, et le cas échéant par désacétalisation de la fonction acétal du groupement B un deuxième composé intermédiaire (X) ou (XIII), dans lequel X représente Cl, obtenu par substitu-

tion du groupe OH du premier composé intermédiaire par le chlore, et un troisième composé intermédiaire (XI) ou (XIV), dans lequel X représente CN, obtenu par substitution du chlore du deuxième composé intermédiaire par le groupe CN, puis en transformant le troisième composé intermédiaire en un composé de formule (I), soit directement dans le cas où Y est le groupement

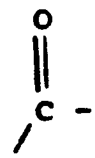

soit dans le cas où Y représente le groupement B, avec désacétalisation de ce dernier groupement.

Selon une deuxième forme de mise en oeuvre de ce même procédé, on effectue la transformation des composés de formule (IV) en composés de formule (I), en formant des composés intermédiaires successifs de formule

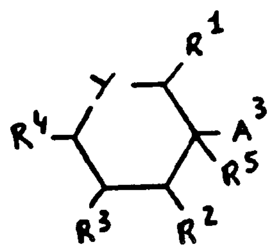

(IX), (X), (XII), (XIII),

dans lesquelles Y représente, soit le groupement B défini dans la revendication 1, soit un groupement

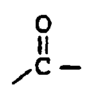

,

et $A^3$ représente un groupement

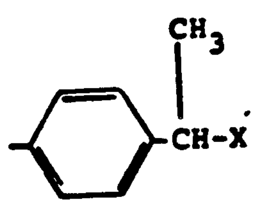

dans lequel X représente OH ou Cl, les significations de Y et X pour chaque formule étant telles qu'indiquées dans le tableau suivant:

| Y = | | B | 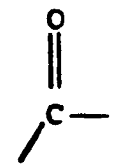 |
|---|---|---|---|
| X = | OH | (IX) | (XII) |
| | Cl | (X) | (XIII) |

à savoir, un premier composé intermédiaire (IX) ou (XII), dans lequel X représente OH, obtenu par réduction de la fonction cétone du groupement $A^1$, et un deuxième composé intermédiaire (X) ou (XIII), dans lequel X représente Cl, obtenu par substitution du groupe OH du premier composé intermédiaire par le chlore en transformant, ensuite, le cas échéant, le composé (XIII) en composé (X) par acétalisation de la fonction cétone Y, puis en transformant le composé (X) intermédiaire en un composé de formule (I) par une réaction de Grignard selon laquelle on fait réagir le dérivé organomagnésien du composé (X) avec l'anhydride carbonique et on hydrolyse le produit d'addition ainsi formé, en effectuant en outre, une désacétalisation du groupement B, de manière à obtenir le produit final (I).

Selon une troisième forme de mise en oeuvre du même procédé, on effectue la transformation des composés de formule (IV) en composés de formule (I) en formant un composé intermédiaire de formule

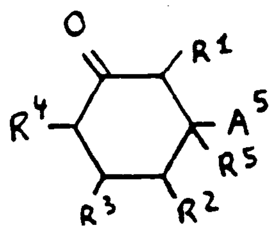

(XV)

dans laquelle $A^5$ représente un groupement

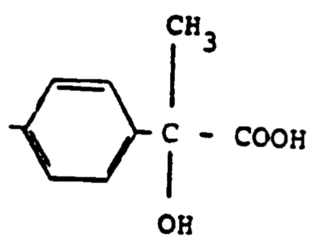

et $R^1$, $R^2$, $R^3$, $R^4$ et $R^5$ sont tels que définis dans la revendication 1, ce composé intermédiaire étant obtenu par réaction du composé (IV) avec le chloroforme et un hydroxyde de métal alcalin, en présence d'une quantité catalytique de chlorure de benzyltriéthylamine, puis désacétalisation du groupement B du composé (IV) par hydrolyse en milieu aqueux acide, et en soumettant le composé intermédiaire (XV) ainsi obtenu à une hydrogénolyse de façon à former le produit (I).

Enfin, selon une quatrième forme de mise en oeuvre du procédé, on effectue la transformation des composés de formule (IV) en composés de formule (I), en faisant d'abord réagir le composé (IV) avec le cyanotriméthylsilane, en présence d'une quantité catalytique d'iodure de zinc, puis en soumettant le produit de cette réaction à une hydrolyse en milieu aqueux acide, de manière à provoquer la désacétalisation du groupement B, le tout de façon à former un composé intermédiaire du type cyanohydrine de formule

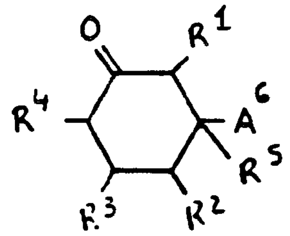

(XVI)

dans laquelle $A^6$ représente un groupement

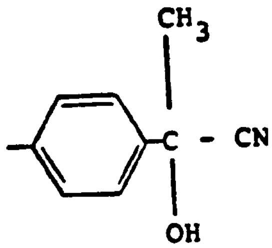

et $R^1$, $R^2$, $R^3$, $R^4$ et $R^5$ sont tels que définis dans la revendication 1, et en soumettant le composé intermédiaire (XVI) ainsi obtenu à une hydrolyse et hydrogénolyse de façon à former le produit final (I).

Ce procédé implique donc l'utilisation de composés ayant la formule générale (IV) susmentionnée, c'est à dire de l'(éthylènedioxo-3,3 cyclohexyl)-4 acétophénone et de dérivés de celle-ci, comme produit de départ pour la préparation de l'acide [(oxo-3 cyclohexyl)-4 phényl]-2 propionique et de ses dérivés.

Les composés de formule (IV) sont des composés nouveaux utiles comme produits de départ pour la préparation des composés de formule (I), par le procédé susmentionné et ils peuvent, bien entendu, être également utiles à d'autres fins.

Un premier procédé de préparation des composés de formule (IV), conforme à l'invention, est caractérisé en ce que:

a) on prépare le dérivé organomagnésien de la p-bromoacétophénone dioxolane de formule

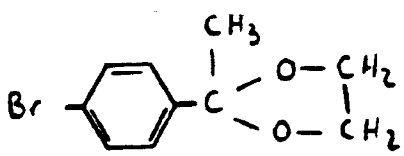

(V)

et on fait réagir ce dérivé organomagnésien avec un composé de formule

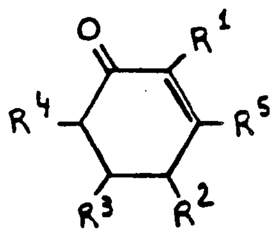

(VIa)

dans laquelle $R^1$, $R^2$, $R^3$, $R^4$ et $R^5$ ont la signification indiquée dans la revendication 1, de façon à obtenir un composé de formule

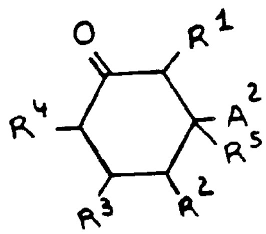

(III)

dans laquelle $A^2$ représente le groupement

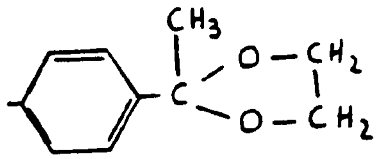

et,

b) on transforme le composé de formule (III) en un composé de formule (IV).

Un deuxième procédé de préparation, selon l'invention, des composés de formule (IV), applicable dans le cas particulier où, dans cette formule, $R^5$ représente l'hydrogène, est caractérisé en ce que:

a) on prépare le dérivé organomagnésien de la p-bromoacétophénone dioxolane de formule

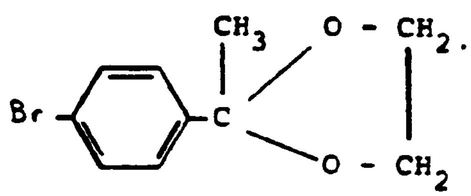

(V)

et on fait réagir ce dérivé organomagnésien avec un composé de formule

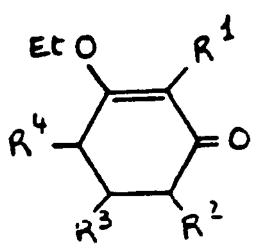

(VI)

dans laquelle Et représente le groupe éthyle et $R^1$, $R^2$, $R^3$, $R^4$ ont la signification indiquée dans la revendication 1, de façon à obtenir un composé de formule

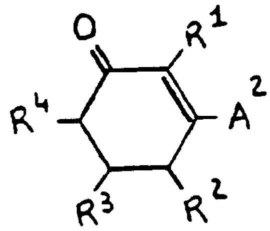

(II)

dans laquelle $A^2$ représente le groupement

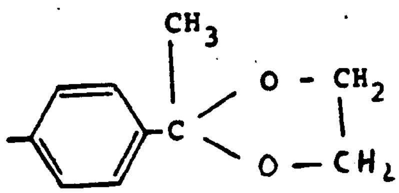

b) on réduit le composé de formule (II), de façon-à obtenir un composé de formule

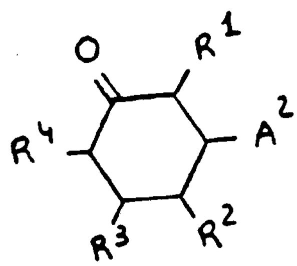

(IIIa)

et,

c) on transforme le composé de formule (IIIa) en un composé de formule (IV) dans laquelle $R^5$ représente l'hydrogène.

On peut effectuer la transformation du composé de formule (III) ou (IIIa), en composé de formule (IV), dans les deux procédés gui viennent d'être mentionnés, soit en une seule opération de transacétalisation catalysée par la présence d'un acide de Lewis et d'éthylène-glycol, soit en transformant d'abord, par désacétalisation du groupement $A^2$ le composé de formule (III) ou (IIIa) en un composé de formule

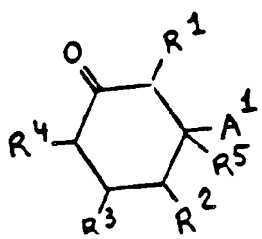

(VII)

puis en transformant ce dernier composé en un composé de formule (IV) par acétalisation de la fonction cétone du groupement cyclohexyle.

Un troisième procédé de préparation, selon l'invention, des composés de formule (IV), applicable, lui aussi, dans le cas particulier où $R^5$ représente l'hydrogène, est caractérisé en ce que:

a) on prépare le dérivé organomagnésien de la p-bromoacétophénone dioxolane de formule

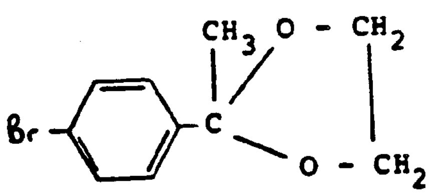

(V)

et on fait réagir ce dérivé organomagnésien avec un composé de formule

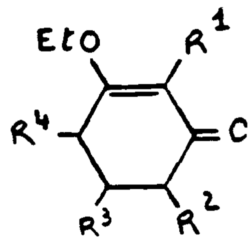

(VI)

dans laquelle Et représente le groupe éthyle et $R^1$, $R^2$, $R^3$, $R^4$ ont la signification indiquée dans la revendication 1, de façon à obtenir un composé de formule

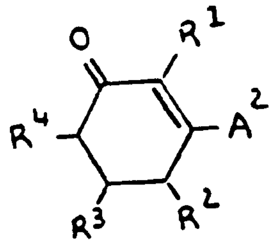

(II)

dans laquelle $A^2$ représente le groupement

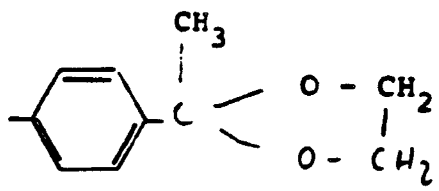

b) on transforme, par désacétalisation du groupement $A^2$, le composé de formule (II) en un composé de formule

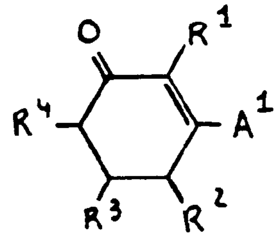

(VIII)

c) on réduit le composé de formule (VIII) de façon à obtenir un composé de formule

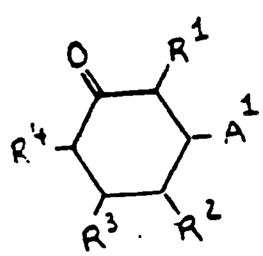

(VIIa)

et,

d) on transforme ce dernier composé en un composé de formule (IV), dans laquelle $R^5$ représente l'hydrogène, par acétalisation de la fonction cétone du groupement cyclohexyle.

Conformément à un quatrième procédé de préparation, selon l'invention, des composés de formule (IV), on fait réagir, par une réaction de Friedel-Crafts, un composé de formule

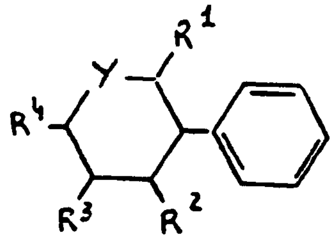

(XVII)

dans laquelle $R^1$, $R^2$, $R^3$, et $R^4$ ont la signification indiquée plus haut et Y représente, soit le groupement B défini dans la revendication 1, soit un groupement $\rangle C = O$, avec du chlorure d'aluminium et un composé choisi parmi le chlorure d'acétyle et l'anhydride acétique, et l'on soumet ensuite le produit de cette réaction à une hydrolysé suivie d'une acétalisation, de façon à former le produit final de formule (IV).

Les procédés de préparation conformes à l'invention comprennent notamment les suites de réactions illustrées à titre d'exemple au dessin annexé dans lequel:

La Fig. 1 illustre les procédés de préparation des composés de formule (IV);

La Fig. 2 illustre la préparation des composés de formule (I) à partir des composés de formule (IV);

La Fig. 3 est une autre forme de représentation schématique des suites de réactions de la Fig. 1, destinée à mettre particulièrement en évidence les caractéristiques stéréochimiques des différents composés; et

La Fig. 4 est une forme de représentation schématique, analogue à celle de la Fig. 3, des suites de réactions de la Fig. 2.

Le produit de départ de formule (V), à savoir la p-bromoacétophénone dioxolane, est un composé connu, facilement préparable, par exemple à partir de la p-bromoacétophénone, comme décrit dans un article publié par A.K. Zarkadis et al. (Chem.Berichte 188, 1183 (1985)).

Les composés de formule (III) ((oxo-3-cyclohexyl)-4 acétophénone dioxolane et ses dérivés substitués par un ou plusieurs groupes alkyles sur le groupement cyclohexyle) peuvent être formés en une seule étape, avec un rendement élevé, par réaction de Grignard entre la p-bromoacétophénone dioxolane et la cyclohexen-2-one ou ses dérivés substitués par des groupes alkyles, c.à.d. les composés de formule (VIa). Cette réaction de Grignard comprend l'addition de l'organomagnésien sur la cétone insaturée, selon la réaction de Michael, catalysée par exemple par Cu Cl ou Cu $(OAc)_2$ (Etape 1).

Les composés de formule (III) peuvent être également formés, avec un excellent rendement, par hydrogénation catalytique des composés de formule (II) dans des conditions permettant l'hydrogénation sélective de la double liaison du groupement cyclohexène sans réduction de la fonction carbonyle. A cet effet, on peut, par exemple, effectuer l'hydrogénation catalytique en milieu EtOH/NaOH en utilisant comme catalyseur le palladium sur carbone (Pd/C) (Etape 3).

Les composés de formule (II) peuvent être formés, avec un très bon rendement, par réaction de Grignard, entre la p-bromoacétophénone dioxolane (V) et l'éthoxy-3-cyclohéxèn-2 one ou ses dérivés substitués alkylés, c.à.d. les composés de formule (VI) (Etape 2). A cet effet, on peut effectuer la condensation des composés (V) et (VI) dans tout solvant approprié, tel que l'éther ou le tétrahydrofuranne (THF). Après l'hydrolyse de la réaction de Grignard et distillation du solvant d'extraction, on obtient une huile suffisamment pure pour être utilisée telle quelle dans l'étape suivante de la synthèse, sans isolement du composé (II). Cependant, ce dernier composé peut être également obtenu par cristallisation à partir de cette huile, par exemple en utilisant un mélange de THF et d'hexane pour provoquer la cristallisation du composé (II).

Pour obtenir les composés de formule (IV) à partir des composés de formule (III), on peut soit effectuer une transacétalisation accélérée par la présence d'éthylène-glycol et d'un acide de Lewis (Etape 4), soit passer par l'intermédiaire des composés de formule (VII). Dans ce dernier cas, on effectue d'abord une désacétalisation du groupement $A^2$ (c.à.d. une déprotection de la fonction cétonique de ce groupement) pour former les composées (VII) à partir des composés (III) (Etape 5), puis une acétalisation de la fonction cétonique de la cyclohexanone, c.à.d. une réaction de protection régiosélective de cette fonction, pour former les composés (IV) à partir des composés (VII) (Etape 6). Il est à remarquer que, par désacétalisation des composés (IV) on peut obtenir les composés (VIII) (Etape 9).

Pour former les composés de formule (VIII), c.à.d. l'(oxo-3- cyclohexen-1-yl)-4 acétophénone et ses dérivés substitués alkylés à partir des composés de formule (II) (Etape 7), on procède à une désacétalisation du groupe $A^2$. A cet effet, on peut effectuer une hydrolyse acide des composés (II).

Par hydrogénation catalytique des composés (VIII), qui peut être effectuée de la même façon que la réduction des composés (II) en composés (III), on peut obtenir les composés de formule (VII) correspondants (Etape 8).

On comprend donc que les différentes voies de synthèse mettant en oeuvre les suites de réactions illustrées à la Fig. 1 permettent de préparer les composés de formule (IV) à partir de la p-bromoacétophénone dioxolane (composé (V) de manière sélective, avec des rendements excellents.

Les composés intermédiaires (II), (III), (VII) et (VIII) formés au cours de ces nouvelles voies de synthèse sont des composés nouveaux gui peuvent être, bien entendu, utilisés non seulement en tant que composés intermédiaires dans les procédés de préparation, selon l'invention, des composés de formule (IV), mais également à d'autres fins.

Ces composés intermédiaires (II), (III), (VII) et (VIII) font également l'objet de l'invention.

La préparation de l'acide [(oxo-3-cyclohexyl)-4 phényl]-2 propionique et de ses dérivés, de formule générale (I), à partir des composés de formule (IV), selon l'invention, comprend la formation successive de trois composés intermédiaires (IX), (X) et (XI) ou bien (XII), (XIII) et (XIV), comme illustré aux Figs 2 et 4.

A cet effet, on forme d'abord les dérivés (IX) ou (XII) dans lesquels la fonction X du groupe $A^3$ est une fonction alcool, par réduction de la fonction cétonique du groupe $A^1$, de manière connue en soi.

On forme ensuite les dérivés (X) ou (XIII), dans lesquels la fonction X du groupe $A^3$ est une fonction chlorure d'alkyle, par chloruration de l'alcool (IX) ou (XII) correspondant, par exemple par traitement de ce dernier par le chlorure de thionyle. Après quoi, on substitue la fonction chloro des composés (X) ou (XIII) par la fonction cyano de manière à former les dérivés du type nitrile (XI) ou (XIV) correspondants. A cet effet, on peut, par exemple, traiter les composés (X) ou (XIII) par un cyanure alcalin, tel que Na CN, par chauffage à reflux dans un solvant approprié tel que le diméthylformamide (DMF).

Finalement, on hydrolyse le groupe cyano des composés (XI) ou (XIV), de manière à former la fonction acide du groupement $A^4$ du produit final (I). A cet effet, on opère avantageusement l'hydrolyse en milieu basique.

Les composés (X), (X), (XI), (XII), (XIII) et (XIV) sont des composés nouveaux qui sont également l'objet de l'invention.

Les exemples non limitatifs suivants illustrent les composés nouveaux et la mise en oeuvre des procédés, selon l'invention:

## EXEMPLE 1

### (Oxo-3 cyclohexén-1-yl)-4 acétophénone -éthylacétal

(Formule (II) $R^1 = R^2 = R^3 = R^4 = H$)

A une solution du magnésien dérivant de la p-bromoacétophénone dioxolane (V), préparée à reflux (60°C), à partir de 20 g (82,5 mmole) du composé (V), 2.2 g de Mg (90,3 mmole) et 160 ml de THF, on ajoute goutte à goutte une solution d'éthoxy-3 cyclohexén-2-one (VI) (12,7 g, 90,6 mmole) dans 50 ml de THF. L'agitation est poursuivie pendant 3h. en maintenant la température à 60°C. Après l'hydrolyse in situ à 5°C par HCl IN (90 ml) et les traitements usuels, on obtient 23,5 g d'un produit huileux dont l'analyse par chromatographie en phase gazeuse (GC) indique 82 % de composé (II) et 10 % de composé (VI). On distille les impuretés sous vide, le résidu cristallise spontanément. On obtient ainsi 19 g du composé (II) (P.F. = 85-87°c recristallisé dans THF/hexane : 1/2).

IR($CH_2Cl_2$) : 3200(w), 2960(sh,m), 2942(m), 2880(m), 1650(s), 1595(s), 1555(w), 1500(w), 1445(w), 1405(m), 1385(w), 1360(w), 1340(m), 1320(m), 1240(s), 1175(m), 1140(m), 1120(sh), 1110(sh), 1080(m), 1050(m), 1020(w), 1005(sh), 985(m), 945(m), 940(sh), 920(sh), 880(m), et 810(m) $cm^{-1}$.

UV(EtOH) : 286 nm

$^1$H-RMN ($CDCl_3$, 360 MHz): 7.54 (s, 4H, H-arom.); 6.43 (s, 1H, H-oléf.); 4.07 (m, 2H dioxolane); 3.78 (m, 2H, dioxolane); 2.80 (dt, J=6 et 1.6, 2H, H-C4"); 2.50 (t, J=6, 2H, H-C6"); 2.17 (m, 2H, H-C5"); et 1.67 ppm (s, 3H, Me).

MS(IE) : 258 M, (2 %); 244 (16.4 %); 243 (M-méthyl, 100 %); 199 (M-dioxolane, 15,5 %).

EXAMPLE 2

(oxo-3 cyclohexyl)-4 acétophénone -éthylacétal

(Formule (III) $R^1 = R^2 = R^3 = R^4 = R^5 = H$)

A. Par réduction du composé (II)

Une solution contenant 13,15 g (50.9 mmole) de (oxo-3 cyclohexén-1-yl)-4 acétophénone -éthylacétal et 32 ml NaOH (32 %) dans 120 ml d'éthanol est hydrogénée en présence de 1,3 g de Palladium 3 % sur charbon, à température ambiante et sous 4 atmosphères d'hydrogène. Après 8 h., on filtre le catalyseur, on évapore le solvant et on ajoute 200 ml d'acétate d'éthyle, 50 ml $H_2O$ déminéralisée et 2 ml HCl (en solution aqueuse à 37 %), successivement. La phase organique est séchée sur $Na_2SO_4$, filtrée et distillée. On obtient ainsi 12,6 g du composé (III).

IR($CH_2Cl_2$) : 1710 $cm^{1}$

$^1$H-RMN ($CDCl_3$, 360 MHz) 7.45 (d, J=8, 2H, H-C2' et H-C6' arom.); 7.20 (d, J=8, 2H, H-C3'et H-C5' arom.); 4.07 (m, 2H, dioxolane); 3.82 (m, 2H, dioxolane); 3.04 (m, 1H H-C1" benzylique); 2.6-1.7 (m, 8H, H-cyclohexanone) et 1.67 (s, 3H, méthyl).

B. Par une réaction de Grignard, à partir de la p-bromoacétophénone dioxolane (V).

On refroidit à -20°C le réactif de Grignard obtenu à partir de 17 g (70 mmole) de la p-bromoacétophénone dioxolane et 1,9 g de Mg (78,19 mmole) dans 80 ml de THF. Puis on y ajoute 0,6 g de CuCl et une solution de 6.2 g (64 mmole) de la cyclohexén-2-one dans 20 ml de THF. On laisse réagir à -20°C pendant 30 min., puis 1 h. à température ambiante.

Après l'hydrolyse avec une solution saturée de chlorure d'ammonium (100 ml) et les traitements habituels, on obtient 12,5 g du produit (III).

EXEMPLE 3

(Ethylènedioxo-3,3 cyclohexyl)-4 acétophénone

(Formule (IV) $R^1 = R^2 = R^3 = R^4 = R^5 = H$)

A. Par une transacétalisation du composé (III)

A 13 g (50 mmole) de (oxo-3 cyclohexyl)-4 acétophénone -éthylacétal (III) et 11,8 g d'éthylèneglycol (0,15 mole) dans 100 ml d'acétone, on ajoute 1,5 ml de $BF_3Et_2O$ (48 % dans l'éther), puis on laisse réagir à température ambiante. La réaction est suivie par GC. Après 2 heures, la conversion est complète. On distille le solvant et on reprend le résidu dans 100 ml d'acétate d'éthyle. On lave successivement avec 30 ml $NaHCO_3$ (10 %) et 50 ml $H_2O$ déminéralisée. La phase organique est séchée sur $Na_2SO_4$, on filtre et on distille le solvant. On obtient 13,1 g d'un produit huileux dont l'analyse GC indique 95 % du produit (IV) et 5 % d'(oxo-3 cyclohexyl)-4 acétophénone (composé de formule (VII). Le produit cristallin pur est obtenu dans un mélange d'acétate d'éthyle/hexane :

IR($CH_2Cl_2$) : 1680(s), 1610(m).

UV(EtOH) : 252 nm

$^1$H-RMN ($CDCl_3$, 300 MHz):

: 7.92 (d, J=8, 2H, H-C2' et H-C6' arom.); 7.32 (d, J=8, 2H, H-C3' et H-C5' arom.); 4.0 (bs, 4H, dioxolane); 2,96 (t,t, 1H, H-benzylique); 2.60 (s, 3H, acétyl) et 1.9-1.4 ppm (m, 8H, cyclohexane).

MS(IE) : 260 (M, 25 %), 217 (M-acétyl, 100 %), 201, 188, 131, 113, 100, 99 (90 %), 86 m/e.

B. Par acétalisation du composé (VII)

A 2 g (9,25 mmole) d'(oxo-3 cyclohexyl)-4 acétophénone (VII) et 1,4 g (22,5 mmole) d'éthylèneglycol dans 10 ml de $CH_2Cl_2$, on ajoute 50 ul de $BF_3$ $Et_2O$ (48 % dans l'éther), et on laisse réagir à température ambiante. Après 1 heure, l'acétalisation est quantitative. Les traitements usuels conduisent à 2,3 g du produit (IV).

EXEMPLE 4

(oxo-3 cyclohexyl)-4 acétophénone

(Formule (VII) $R^1 = R^2 = R^3 = R^4 = R^5 = H$)

A. Désacétalisation du composé (III)

On laisse réagir une solution de 10 g (38,5 mmole) de (oxo-3 cyclohexyl)-4 acétophénone -éthylacétal (III) dans 40 ml d'acétone, en présence d'une quantité catalytique de HCl, pendant 2 h. à la température ambiante. Après distillation du solvant, le produit est repris dans 80 ml d'acétate d'éthyle, on lave avec une solution de 10 % de NaH $CO_3$ (20 ml) et on sèche la phase organique sur $Na_2SO_4$. On élimine le solvant par distillation sous vide partiel pour obtenir 8 g du produit (VII). P.F. 57 - 59°c.

IR($CH_2$) : 1710(s), 1682cm$^{1}$(s), 1610(m).

UV (EtOH) : 250 nm

$^1$H-RMN ($CDCl_3$, 360 MHz):

: 7.45 (d, J=8, 2H, H-C1' et H-C6' arom.); 7.35 (d, J=8, 2H, H-C3' et H-C5' arom.); 3.10 (m, 1H, H-benzylique); 2.60 (s, 3H, acéthyl) et 2.7 - 1.8 (m, 8H, cyclohexanone).

B. Déprotection du composé (IV)

On procédé comme précédemment, dans l'acétone en présence de HCl (catalytique), le rendement en produit (VII) isolé est de 93 %.

C. Réduction catalytique du composé (VIII)

On opère dans les conditions de la réduction catalytique, propre aux cétones insaturées de la même manière que pour la réduction du composé (II) en composé (III). Le produit (VII) est obtenu à partir du composé (VIII) avec un très bon rendement.

EXEMPLE 5

(oxo-3 cyclohexén-1-yl)-4 acétophénone

(Formule (VIII) $R^1 = R^2 = R^3 = R^4 = H$)

17 g (65,9 mmole) d'oxo-3 cyclohexén-1-yl)-4 acétophénone -éthylacétal (II) dans 25 ml d'acétone en présence de HCl catalytique (0,03 ml) sont maintenus à la température ambiante pendant 2 h. Le solvant est distillé et le résidu est repris dans 100 ml d'acétate d'éthyle. La solution organique est lavée avec 30 ml $NaHCO_3$ (10 %) et est séchée sur $Na_2SO_4$. Après la distillation du solvant, on obtient 13,3 g du produit (VIII) qui cristallisent spontanément. P.F. = 91-93°c (recristallisé dans THF/hexane).

IR($CH_2Cl_2$) : 1682(s); 1675(s) et 1603cm$^{1}$(m)

UV(EtOH) : 290 nm

$^1$H-RMN ($CDCl_3$, 360 MHz)

: 8.0 (d, J=8.2, 2H, H-C2' et H-C6' arom.); 7.64 (d, J=8.2, 2H, H-C3' et H-C5' arom.); 6.47 (s, 1H, H-oléf.); 2.8 (dt, J=6 et 1.4, 2H, J-C4"); 2.65 (s, 3H, acétyl); 2.52 (t, J=6, 2H, H-C6"); 2.2 (m, 2H, H-C5").

EXEMPLE 6

[(Ethylènedioxo-3,3 cyclohexyl)-4 phényl]-1 éthanol

(Formule (IX) $R^1 = R^2 = R^3 = R^4 = R^5 = H$ ;

Y = 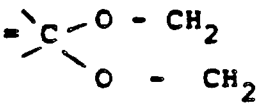

X = OH)

10 g du composé (IV) (38.46 mmole) dans 40 ml de MeOH, sont réduits par 2,4 g (63 mmole) de $NaBH_4$, à température ambiante pendant 1h 30. Le mélange est hydrolysé par une solution diluée de HCl à 5°c. La phase organique (extrait acétate d'éthyle) est lavée à l'eau. Le résidu d'évaporation du solvant organique conduit à 9,8 g du produit (IX).

| | |
|---|---|
| IR(Film) | : 3450 $cm^{-1}$(bs), 1600(v). |
| $^1$H-RMN ($CDCl_3$) | : 1.52 (d, 3H, $CH_3$); 4.90 (q, 1H, H-C1). |

Les déplacements chimiques des autres protons du composé (IX) sont identiques à ceux du produit (IV).

EXEMPLE 7

[(oxo-3 cyclohexyl)-4 phényl]-1 éthanol

(Formule (XII) $R^1 = R^2 = R^3 = R^4 = R^5 = H$ ; Y = >C = O ; X = OH)

La déprotection de la fonction acétal du composé (IX) en fonction cétone du composé (XII) s'effectue dans l'acétone en présence d'une quantité catalytique d'acide chlorhydrique.

IR : 3450(bs), 1710(s).

$^1$H-RMN ($CDCl_3$)

: 7.37 (d, 2H, H-C2' et H-C6'); 7.22 (d, 2H, H-C3' et H-C5'); 4.9 (q, 1H, H-C1); 3.0 (m, 1H, H-benzylique); 2.57-1.75 (m, 8H, cyclohexanone) et 1.5 (d, 3H, $CH_3$).

EXEMPLE 8

[(Ethylènedioxo-3,3 cyclohexyl)-4 phényl]-1 chloro-1 éthane

(Formule (X) $R^1 = R^2 = R^3 = R^4 = R^5 = H$ ;

Y = 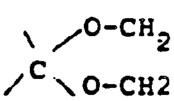

X = Cl)

Une solution du composé (IX) (8 g, 30,5 mmole) dans 30 ml de $CH_2Cl_2$ est traitée par le chlorure de thionyle (2,5 ml ; 34 mmole) en présence de pyridine pendant 2 h. à 10°C. Le mélange réactionnel est neutralisé par une solution de bicarbonate, et la phase organique est séchée sur $Na_2SO_4$. On obtient 8,2 g du produit (X).

$^1$H-RMN ($CD1_3$)

: 7.4 (d, 2H) et 7.22 (d, 2H) les H-arom.; 5.10 (q, 1H, H-Cl); 4.0 (s, 4H, dioxolane); 2.89 (m, H, H-benzylique), 2.6 - 1.3 (m, 8H, cyclohexane) et 1.85 (d, 3H, $CH_3$).

EXEMPLE 9

[(oxo-3 cyclohexyl)-4 phényl]-1 chloro-1 éthane

(Formule (XIII) $R^1 = R^2 = R^3 = R^4 = R^5 = H$ ; Y = > C = O ; X = Cl)

Le produit (XIII) est obtenu quantitativement à partir du composé (X) dans les conditions de déprotections standard.

IR ($CH_2Cl_2$): 1710(s), 1600(w).

$^1$H-RMN ($CDC1_3$)

: 3.03 (m, 1H, H-benzylique), 2.7 - 1.6 (m, 8H, H-cyclohexanone)

EXEMPLE 10

[(Ethylènedioxo-3,3 cyclohexyl)-4 phényl]-2 propionitrile

(Formule (XI) $R^1 = R^2 = R^3 = R^4 = H$ ;

Y = 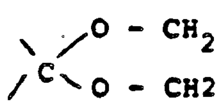

X = CN)

12 g (42,8 mmole) du composé (X) et 6,5 g (0,132 mole) de NaCN dans 30 ml de DMF sont portés à reflux (18 h.). Après hydrolyse et extraction à l'acétate d'éthyle, l'évaporation du solvant organique aboutit à 8,5 g du produit (XI).

EXEMPLE 11

[(oxo-3 cyclohexyl)-4 phényl]-2 propionitrile

(Formule XIV ; $R^1 = R^2 = R^3 = R^4 = R^5 = H$ ; Y = > C = O ; X = CN)

La déprotection de l'acétal (XI) en cétone (XIV) est opérée dans l'acétone en présence d'une quantité catalytique d'acide chlorhydrique.

$IR(CH_2Cl_2)$ : 2220(w), 1710(s)

$^1H$-RMN($CDCl_3$)

: est caractérisé par les δ à 1.67 (d, 3H, $CH_3$) et 3.90 (q, 1H, H-C1).

EXEMPLE 12

Acide [(oxo-3 cyclohexyl)-4 phényl]-2 propionique

(Formule (I) ; $R^1 = R^2 = R^3 = R^4 = R^5 = H$)

A. A partir de la propionitrile (XI)

12 g du composé (XI) (44 mmole), 20 ml KOH (50 %) dans 20 ml EtOH sont portés à reflux pendant 4 h. Le mélange réactionnel est débarrassé de composés non carboxyliques par extraction à l'acétate d'éthyle. La phase aqueuse est acidifiée à pH1 et extraite à nouveau plusieurs fois à l'acétate d'éthyle. L'évaporation de ce solvant conduit à 9 g du produit final (I).

IR(KBr) : 3200(b), 1700(bs), 1720(sh).

$^1H$-RMN ($CDCl_3$)

: 7.30 (d, J=9, 2H, H-C2' et H-C6'); 7.20 (d, J=9, 2H, H-C3' et H-C5'); 3.75 (q, J=7.2, 1H, H-C2); 301 (t,t, J=11.5 et 4, 1H, H-C1" benzylique); 2.6 - 1.6 (m, 8H, cyclohexanone) et 1.55 (t, J=7.2, 3H, H-C3).

B. Via l'acide [(oxo-3 cyclohexyl)-4 phényl], hydroxy-2 propionique

(Composé intermédiaire de formule (XV) dans laquelle $R^1 = R^2 = R^3 = R^4 = R^5 = H$)

On traite une solution d'éthylènedioxo-3,3 cyclohexyl)-4 acétophénone (composé (Iv) (5 g; 19.2 mmole) dans 10 ml de $CHCl_3$ et 50 ml de toluène, en présence de chlorure de benzyltriéthylamine, avec 15 ml de NaOH (32%) 18 heures à température ambiante. Le composé cétonique est extrait à acétate d'éthyle, et la phase aqueuse est acidifiée à pH 1. Le produit de formule (XV) est extrait à l'acétate d'éthyle et est soumis à l'hydrogénolyse dans l'éthanol en présence du Palladium sur charbon pour conduire à 2,8 g de produit final (I).

C. Via la cyanohydrine: [(oxo-3 cyclohexyl)-4 phenyl]-2, hydrox-2, propionitrile

(Composé intermédiaire de formule (XVI) dans laquelle $R^1 = R^2 = R^3 = R^4 = R^5 = H$)

A 6 g d'(éthylènedioxo-3,3 cyclohexyl)-4 acétophénone (23 mmole) dans 20 ml $CH_2Cl_2$ refroidi à 0°C, on ajoute 4 ml de cyanotriméthylsilane et quelques cristaux de $ZnI_2$, on laisse réagir 1 heure à 0°C. On soumet ensuite la cyanohydrine (XVI) à une hydrolyse acide et à une hydrogénolyse (effectuées comme indiqué en B. ci-dessus) pour aboutir à 3,2 g du produit (I).

D. Par une réaction de Grignard sur le composé chloré (X)

On refroidit, à 10°C, le réactif de Grignard obtenu à partir de 5 g (17,8 mmole) de composé (X) et 0,55 g(22,9 mmole) de Mg dans THF (ou $Et_2O$) et on fait passer un courant de $CO_2$ dans le milieu réactionnel, tout en maintenant la température entre 5 et 10°C (2 heures). Après les traitements propres aux réactions de Grignard, on obtient 2,7 g de produit (I).

EXEMPLE 13

Préparation de l'(éthylènedioxo-3,3 cyclohexyl)-4 acétophénone (Formule (IV) $R^1 = R^2 = R^3 = R^4 = R^5 =$ H) par une réaction de Friedel-Crafts à partir de phényl-3 cyclohexanone (composé de formule (XVII) dans laquelle $R^1 = R^2 = R^3 = R^4 =$ H et Y = $>$C = O.

A 10 g (57,47 mmole) du composé (XVII) et 4,5 g (57,69 mmole) de chlorure d'acétyle dans 50 ml de cyclohexane, on ajoute 12 g (90 mmole) de chlorure d'aluminium, en 45 minutes, en refroidissant en dessous de 5°C. Après 3 heures d'agitation à cette température, le mélange est hydrolysé sur de la glace et extrait à l'acétate d'éthyle. Les traitements usuels de la réaction de Friedel-Crafts et d'acétalisation conduisent à 6 g du produit (IV).

## Revendications

1. Composés de formule

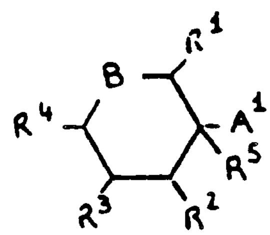

(IV)

dans laquelle $A^1$ représente un groupement

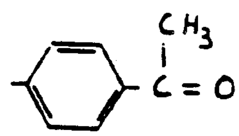

B représente un groupement

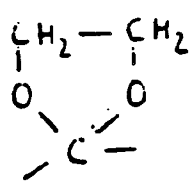

et $R^1$, $R^2$, $R^3$, $R^4$, et $R^5$, qui peuvent être identiques ou différents, représentent chacun l'hydrogène ou un groupe alkyle.

2. Procédé de préparation des composés de formule (IV) selon la revendication 1, caractérisé en ce que:
   a) on prépare le dérivé organomagnésien de la p-bromoacétophénone dioxolane de formule

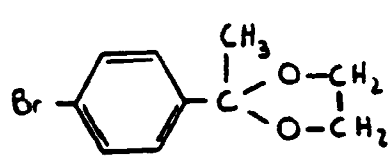

(V)

et on fait réagir ce dérivé organomagnésien avec un composé de formule

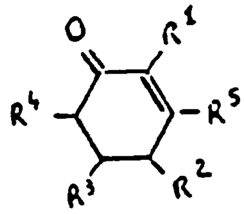

(VI a)

dans laquelle $R^1$, $R^2$, $R^3$, $R^4$ et $R^5$ ont la signification indiquée dans la revendication 1, de façon à obtenir un composé de formule

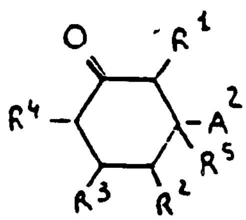

(III)

dans laquelle $A^2$ représente le groupement

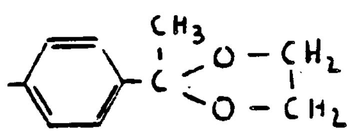

et,

b) on transforme le composé de formule (III) en un composé de formule (IV).

3. Procédé de préparation des composés de formule (IV) selon la revendication 1, dans laquelle $R^5$ représente l'hydrogène, caractérisé en ce que:

a) on prépare le dérivé organomagnésien de la p-bromoacétophénone dioxolane de formule

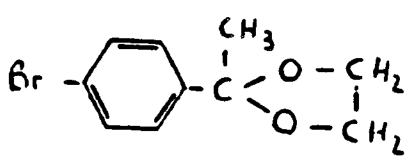

(V)

et on fait réagir ce dérivé organomagnésien avec un composé de formule

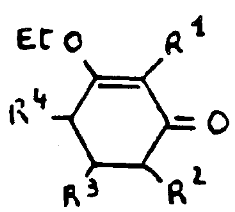

(VI)

dans laquelle Et représente le groupe éthyle et $R^1$, $R^2$, $R^3$, $R^4$ ont la signification indiquée dans la revendication 1, de façon à obtenir un composé de formule

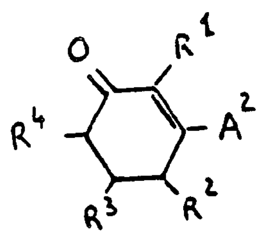

(II)

dans laquelle $A^2$ représente le groupement

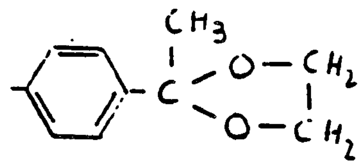

b) on réduit le composé de formule (II), de façon à obtenir un composé de formule

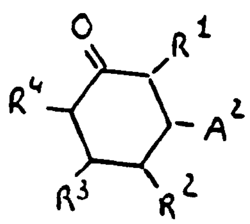

(IIIa)

et,

c) on transforme le composé de formule (IIIa) en un composé de formule (IV) dans laquelle $R^5$ représente l'hydrogène.

**4.** Procédé selon la revendication 2 ou la revendication 3, caractérisé en ce que l'on effectue la transformation du composé de formule (III) ou (IIIa) en composé de formule (IV) en une seule opération de transacétalisation catalysée par la présence d'un acide de Lewis et d'éthylène-glycol.

**5.** Procédé selon la revendication 2 ou la revendication 3, caractérisé en ce que l'on effectue la transformation du composé de formule (III) ou (IIIa) en composé de formule (IV) en transformant, d'abord, par désacétalisation du groupement $A^2$, le composé de formule (III) ou (IIIa) en un composé de formule

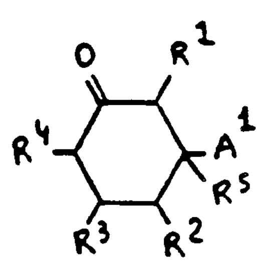

(VII)

puis en transformant ce dernier composé en un composé de formule (IV) par acétalisation sélective de la fonction cétone du groupement cyclohexyle.

**6.** Procédé de préparation des composés de formule (IV) selon la revendication 1, dans laquelle $R^5$ représente d'hydrogène, caractérisé en ce que:

a) on prépare le dérivé organomagnésien de la p-bromoacétophénone dioxolane de formule

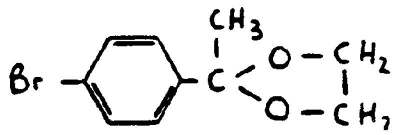

(V)

et on fait réagir ce dérivé organomagnésien avec un composé de formule

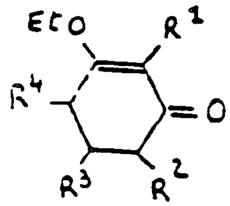

(VI)

dans laquelle Et représente le groupe éthyle et $R^1$, $R^2$, $R^3$, $R^4$ ont la signification indiquée dans la revendication 1, de façon à obtenir un composé de formule

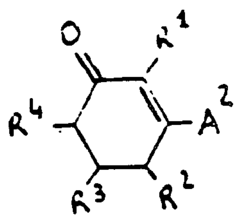

(II)

dans laquelle $A^2$ représente le groupement

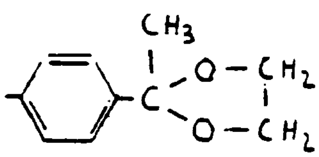

b) on transforme, par désacétalisation du groupement $A^2$, le composé de formule (II) en un composé de formule

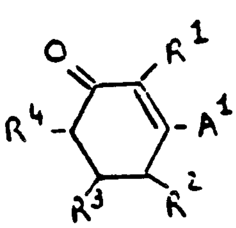

(VIII)

c) on réduit le composé de formule (VIII) de façon à obtenir un composé de formule

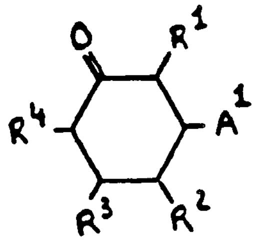

(VIIa)

et,
d) on transforme ce dernier composé en un composé de formule (IV), dans laquelle $R^5$ représente l'hydrogène, par acétalisation de la fonction cétone du groupement cyclohexyle.

7. Procédé de préparation des composés de formule (IV) selon la revendication 1, caractérisé en ce que l'on fait réagir, par une réaction de Friedel-Crafts, un composé de formule

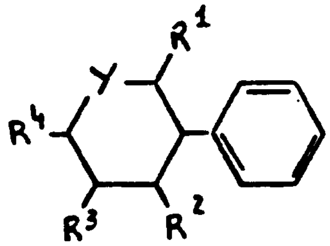

(XVII)

dans laquelle Y représente soit le groupement B défini dans la revendication 1, soit un groupement >C = O, avec du chlorure d'aluminium et un composé choisi parmi le chlorure d'acétyle et l'anhydride acétique, et l'on soumet ensuite le produit de cette réaction à une hydrolyse suivie d'une acétalisation, de façon à former le produit final de formule (IV).

8. Procédé de préparation de l'acide [(oxo-3 cyclohexyl) -4 phényl] - 2 propionique et de dérivés de celui-ci, de formule

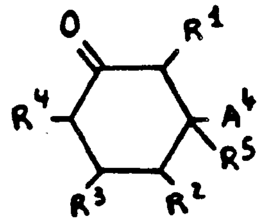

(I)

dans laquelle $A^4$ représente un groupement

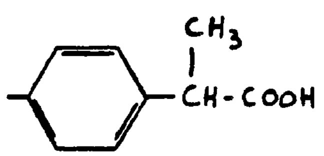

et $R^1$, $R^2$, $R^3$, $R^4$, $R^5$, qui peuvent être identiques ou différents représentent chacun l'hydrogène ou un groupe alkyle, caractérisé en ce que l'on transforme les composés de formule (IV), tels que définis dans la revendication 1, en composés de formule (I).

9. Procédé selon la revendication 8, caractérisé en ce que l'on effectue la transformation des composés de formule (IV) en composés de formule (I) en formant des composés intermédiaires successifs de formule:

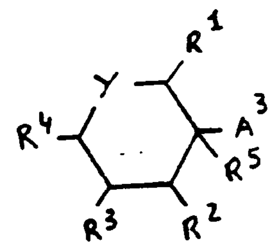

(IX), (X), (XI),
(XII), (XIII), (XIV)

dans lesquelles Y représente, soit le groupement B défini dans la revendication 1, soit un groupement

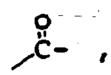

et $A^3$ représente un groupement

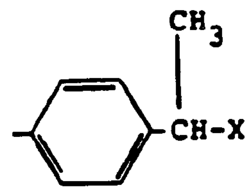

dans lequel X représente OH, Cl ou CN, les significations de Y et X pour chaque formule étant telles qu'indiquées dans le tableau suivant:

| Y = | | B | 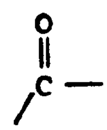 |
|---|---|---|---|
| X = | OH | (IX) | (XII) |
| | Cl | (X) | (XIII) |
| | CN | (XI) | (XIV) |

à savoir, un premier composé intermédiaire (IX) ou (XII), dans lequel X représente OH, obtenu par réduction de la fonction cétone du groupement $A^1$, et le cas échéant par désacétalisation de la fonction acétal du groupement B un deuxième composé intermédiaire (X) ou (XIII), dans lequel X représente Cl, obtenu par substitution du groupe OH du premier composé intermédiaire par le chlore et un troisième composé intermédiaire (XI) ou (XIV), dans lequel X représente CN, obtenu par substitution du chlore du deuxième composé intermédiaire par le groupe CN, puis en transformant le troisième composé intermédiaire en un composé de formule (I), soit directement. dans le cas où Y est le groupement

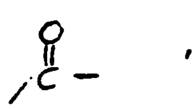

ou bien, dans le cas où Y représente le groupement B, avec désacétalisation de ce dernier groupement.

**10.** Procédé selon la revendication 8, caractérisé en ce que l'on effectue la transformation des composés de formule (IV) en composés de formule (I), en formant des composés intermédiaires successifs de formule

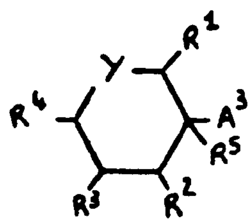

(IX), (X), (XII), (XIII)

dans lesquelles Y représente, soit le groupement B défini dans la revendication 1, soit un groupement

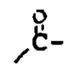

et $A^3$ représente un groupement

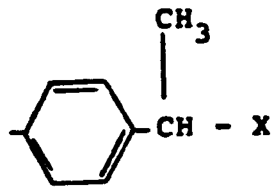

dans lequel X représente OH ou Cl, les significations de Y et X pour chaque formule étant telles qu'indiquées dans le tableau suivant:

| | | | |
|---|---|---|---|
| Y = | | B | 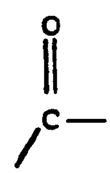 |
| X = | OH | (IX) | (XII) |
| | Cl | (X) | (XIII) |

à savoir, un premier composé intermédiaire (IX) ou (III), dans lequel X représente OH, obtenu par réduction de la fonction cétone du groupement $A^1$, et un deuxième composé intermédiaire (X) ou (XIII), dans lequel X représente Cl, obtenu par substitution du groupe OH du premier composé intermédiaire par le chlore, en transformant, ensuite, le cas échéant, le composé (XIII) en composé (X) par acétalisation de la fonction cétone Y, puis en transformant le composé (X) en un composé de formule (I) par une réaction de Grignard selon laquelle on fait réagir le dérivé organomagnésien du composé (X) avec l'anhydride carbonique et on hydrolyse le produit d'addition ainsi formé, en effectuant en outre, une désacétalisation du groupement B, de manière à obtenir le produit final (I).

**11.** Procédé selon la revendication 8, caractérisé en ce que l'on effectue la transformation des composés de formule (IV) en composé intermédiaire de formule

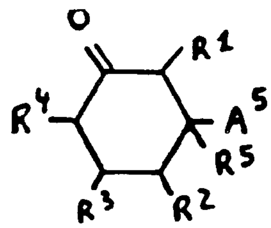

(XV)

dans laquelle $A^5$ représente un groupement

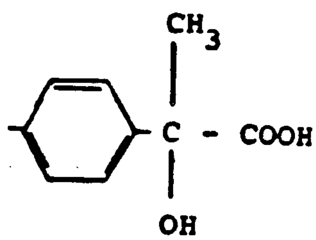

et $R^1$, $R^2$, $R^3$, $R^4$ et $R^5$ sont tels que définis dans la revendication 1, ce composé intermédiaire étant obtenu par réaction du composé (IV) avec le chloroforme et un hydroxyde de métal alcalin, en présence d'une quantité catalytique de chlorure de benzyltriéthylamine, puis désacétalisation du groupement B du composé (IV) par hydrolyse en milieu aqueux acide, et en soumettant le composé intermédiaire (XV) ainsi obtenu à une hydrogénolyse de façon à former le produit (I).

12. Procédé selon la revendication 8, caractérisé en ce que l'on effectue la transformation des composés de formule (IV) en composés de formule (I) en faisant d'abord réagir le composé (IV) avec le cyanotriméthylsilane, en présence d'une quantité catalytique d'iodure de zinc, puis en soumettant le produit de cette réaction à une hydrolyse en milieu aqueux acide, de manière à provoquer la désacétalisation du groupement B, le tout de façon à former un composé intermédiaire du type cyanohydrine de formule

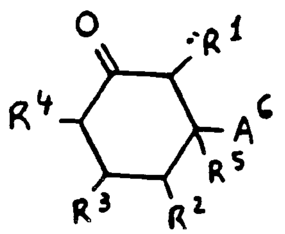

(XVI)

dans laquelle $A^6$ représente un groupement

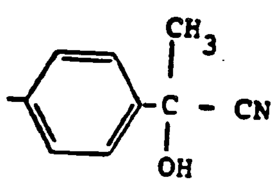

et $R^1$, $R^2$, $R^3$, $R^4$ et $R^5$ sont tels que définis dans la revendication 1, et en soumettant le composé intermédiaire (XVI) ainsi obtenu à une hydrolyse et une hydrogénolyse de façon à former le produit final (I).

13. Composés de formule

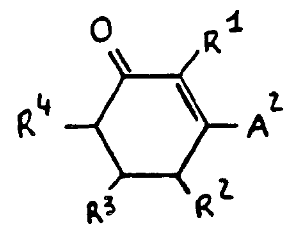

(II)

dans laquelle $A^2$ représente le groupement

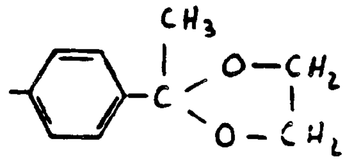

et $R^1$, $R^2$, $R^3$, $R^4$, qui peuvent être identiques ou différents, représentent chacun l'hydrogène ou un groupe alkyle.

**14.** Composés de formule

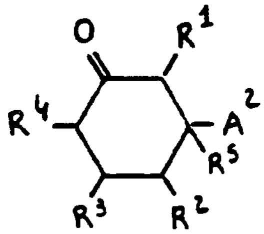

(III)

dans laquelle $A^2$ représente le groupement

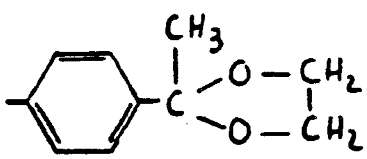

et $R^1$, $R^2$, $R^3$, $R^4$ et $R^5$, qui peuvent être identiques ou différents, représentent chacun l'hydrogène ou un groupe alkyle.

**15.** Composés de formule

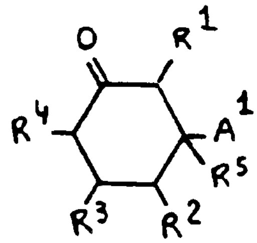

(VII)

dans laquelle $A^1$, $R^1$, $R^2$, $R^3$, $R^4$ et $R^5$ sont tels que définis dans la revendication 1.

**16.** Composés de formule

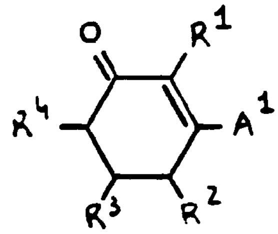

(VIII)

dans laquelle $A^1$, $R^1$, $R^2$, $R^3$ et $R^4$ sont tels que définis dans la revendication 1.

**17.** Composés de formules

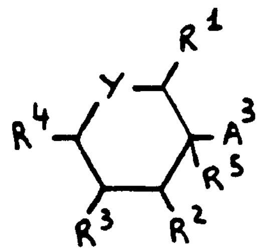

(IX), (X), (XI), (XII), (XIII), (XIV)

dans lesquelles Y représente, soit le groupement B défini dans la revendication 1, soit un groupement >C=O, et $A^3$ représente un groupement

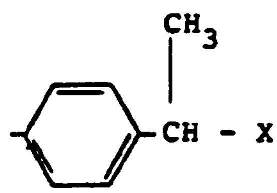

dans lequel X représente OH, Cl ou CN, les significations de Y et X pour chaque formule étant telles qu'indiquées dans le tableau suivant:

| | Y = | B | 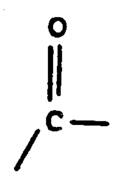 |
|---|---|---|---|
| X = | OH | (IX) | (XII) |
| | Cl | (X) | (XIII) |
| | CN | (XI) | (XIV) |

## Claims

**1.** Compounds of the formula

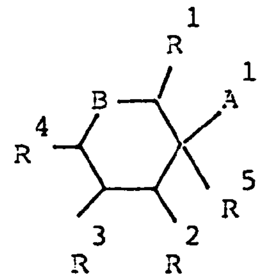

(IV)

in which $A^1$ represents a group

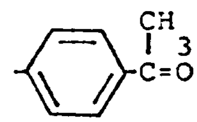

and B represents a group

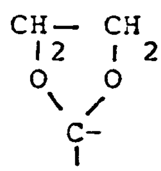

and $R^1$, $R^2$, $R^3$, $R^4$, $R^5$, which can be indentical or different, represent each a hydrogen or an alkyl group.

**2.** A method for the preparation of the compounds of formula (IV) according to Claim 1, characterized in that
a) an organomagnesium derivative of p-bromoacetophenone dioxolane of the formula

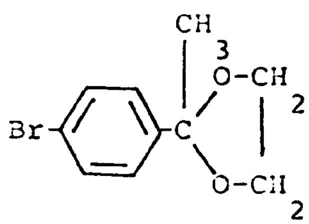

(V)

is prepared and this organomagnesium derivative is reacted with a compound of the formula

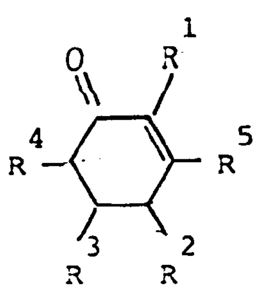

(VIa)

in which $R^1$, $R^2$, $R^3$, $R^4$ and $R^5$ have the meaning indicated in Claim 1, to obtain a compound of the formula

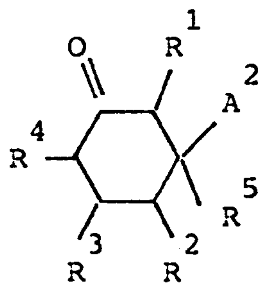

(III)

in which $A^2$ represents the group

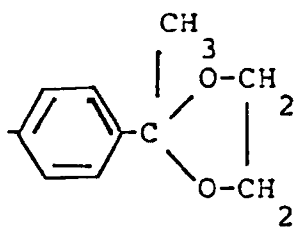

and

b) the compound of formula (III) is transformed into a compound of formula (IV).

**3.** A method for the preparation of the compounds of formula (IV) according to Claim 1, in which $R^5$ represents a hydrogen, characterized in that :

a) an organomagnesium derivative of p-bromoacetophenone dioxolane of the formula

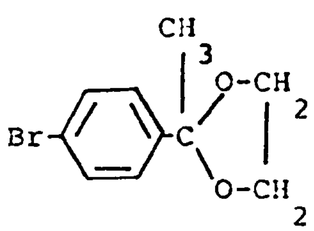

(V)

is prepared and this organomagnesium derivative is reacted with a compound of the formula

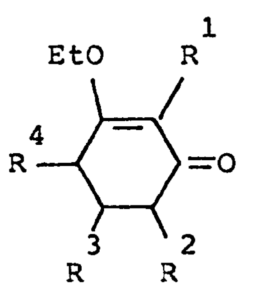

(VI)

in which Et represents the ethyl group and $R^1$, $R^2$, $R^3$, $R^4$ have the meaning indicated in Claim 1, to obtain a compound of the formula

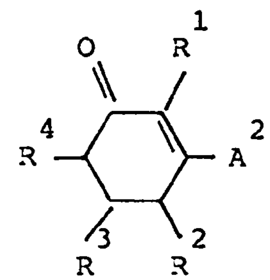

(II)

in which $A^2$ represents the group

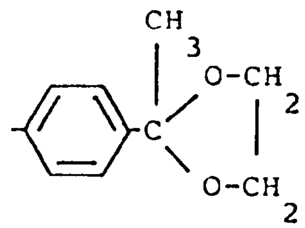

and

b) the compound of formula (II) is reduced to obtain a compound of the formula

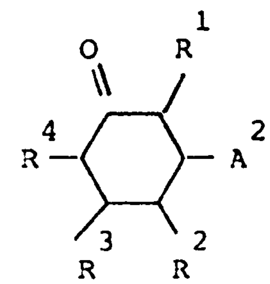

(IIIa)

and

c) the compound of formula (IIIa) is transformed into a compound of formula (IV), in which $R^5$ represents a hydrogen.

**4.** A method according to Claim 2 or Claim 3, characterized in that the transformation of the compound of formula (III) or (IIIa) into a compound of formula (IV) is carried out in a single operation of transacetalisation catalyzed by the presence of a Lewis acid and of ethylene glycol.

**5.** A method according to Claim 2 or Claim 3, characterized in that the transformation of the compound of formula (III) or (IIIa) into a compound of formula (IV) is carried out by transforming first by deacetalisation of the group $A^2$ the compound of formula (III) or (IIIa) into a compound of the formula

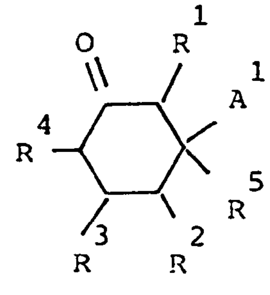

(VII)

and then by transforming the latter compound into a compound of formula (IV) through the selective acetalisation of the ketone function of the cyclohexyl group.

6. A method for the preparation of the compounds of formula (IV) according to claim 1, in which $R^5$ represents a hydrogen, characterized in that :

a) an organomagnesium derivative of p-bromoacetophenone dioxolane of the formula

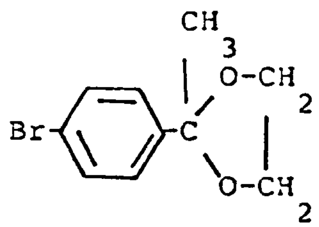

(V)

is prepared and this organomagnesium derivative is reacted with a compound of the formula

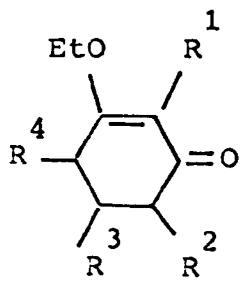

(VI)

in which Et represents the ethyl grour and $R^1$, $R^2$, $R^3$, $R^4$ have the meaning indicated in Claim 1, to obtain a compound of the formula

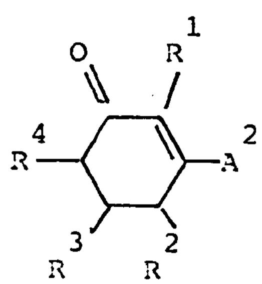

(II)

in which $A^2$ represents the group

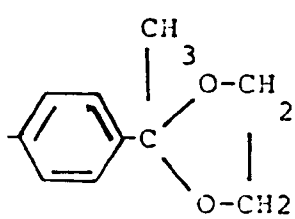

b) the compound of formula (II) is transformed by deacetalisation of the group $A^2$ into a compounds of the formula

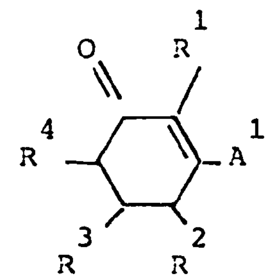

(VIII)

c) the compound of the formula (VIII) is reduced to obtain a compound of the formula

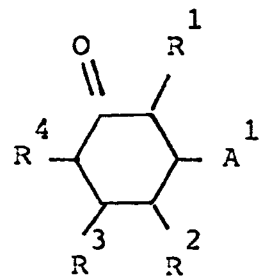

(VIIa)

and

d) the latter compound is transformed into a compound of the formula (IV), in which $R^5$ represents a hydrogen, through the acetalisation of the ketone function of the cyclohexyl group.

7. A method of preparation of compounds of the formula (IV) according to Claim 1, characterized in that a compound of the formula

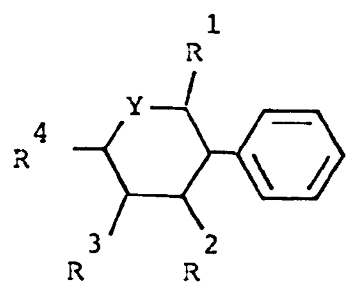

(XVII)

in which Y represents either the group B defined in Claim 1 or a >C=O group, is reacted in a Friedel-Crafts reaction with aluminium chloride and a compound selected from acetyl chloride or acetic anhydride and then, the product of this reaction is subjected to a hydrolysis, followed by an acetalisation, to form the final product of formula (IV).

8. A method of preparation of 2-[4-(3-oxo-cyclohexyl)phenyl] propionic acid and of its derivatives of the formula

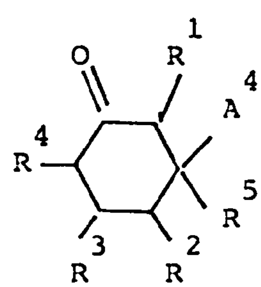

(I)

in which $A^4$ represents a group

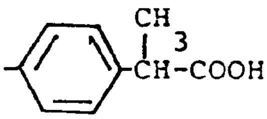

and $R^1$, $R^2$, $R^3$, $R^4$, $R^5$, which can be identical or different, represent each a hydrogen or an alkyl group, characterized in that the compounds of formula (IV), such as defined in Claim 1 are transformed into the compounds of formula (I).

9. A method according to Claim 8, characterized in that the compounds of formula (IV) are transformed into the compounds of formula (I), by forming successive intermediate compounds of the formula

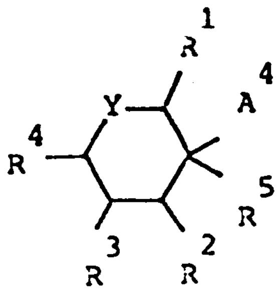

(IX) , (X), (XI)
(XII), (XIII), (XIV)

in which Y represents either the group B defined in Claim 1, or a group

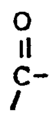

and $A^3$ represents a group

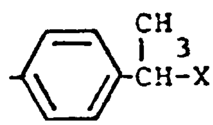

in which X represents OH, Cl or CN, the meaning of Y and X for each formula being that indicated in the following table :

| Y = | | B | 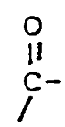 |
|---|---|---|---|
| X = | OH | (IX) | (XII) |
| | Cl | (X) | (XIII) |
| | CN | (XI) | (XIV) |

namely, a first intermediate compound (IX) or (XII) in which X represents OH, obtained by the reduction of the ketone function of the group $A^1$ and, when required, the deacetalisation of the acetal function of the

group B, a second intermediate compound (X) or (XIII) in which X represents Cl, obtained by a substitution of chlorine for the OH group of the first intermediate compound, and a third intermediate compound (XI) or (XIV) in which X represents CN, obtained by the substitution of the CN group for the chlorine of the second intermediate compound, then by transforming the third intermediate compound into a compound of formula (I), either directly in the case where Y is the group

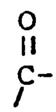

or, in the case where Y represents the group B, with deacetalisation of the latter group.

**10.** A method according to Claim 8, characterized in that a transformation of the compounds of formula (IV) into the compounds of formula (I) is carried out by forming the successive intermediate compounds of the formula

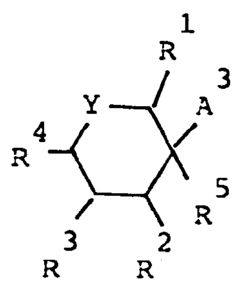

(IX), (X), (XII), (XIII)

in which Y represents either the group B defined in Claim 1 or a group

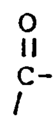

and $A^3$ represents a group

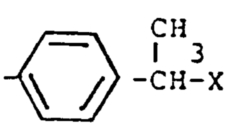

in which X represents OH or Cl, the meaning of Y and X for each formula being that indicated in the following table :

| | | | |
|---|---|---|---|
| Y = | | B | 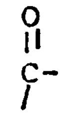 |
| X = | OH | (IX) | (XII) |
| | Cl | (X) | (XIII) |

namely a first intermediate compound (IX) or (XII) in which X represents OH, obtained through the reduction of the ketone function of the group $A^1$, and a second intermediate compound (X) or (XIII) in which X represents Cl, obtained through the substitution of the chlorine for the OH group of the first intermediate compound and afterwards by transforming, when required, the compound (XIII) into the compound (X) through the acetalisation of the ketone function Y, then by transforming the compound (X) into a compound of formula (I) through a Grignard reaction, in which the organomagnesium derivative of compound (X) is reacted with carbon dioxide and the addition product thus formed is hydrolyzed while at the same time a deacetalisation of the group B occurs, to obtain the final product (I).

**11.** A method according to Claim 8, characterized in that the compounds of formula (IV) are transformed into the intermediate product of the formula

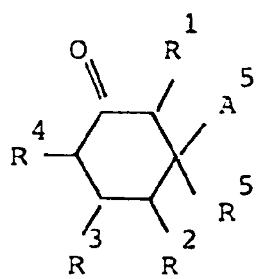

(XV)

in which $A^5$ represents a group

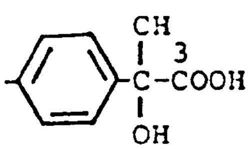

and $R^1$, $R^2$, $R^3$, $R^4$ and $R^5$ are such as defined in Claim 1, this intermediate compound being obtained through the reaction of the compound (IV) with chloroform and an alkali metal hydroxide in the presence of a catalytic amount of benzyltriethylamine chloride, followed by the deacetalisation of the group B of the compound (IV) through hydrolysis in an acidic aqueous medium and by subjecting the intermediate compound (XV) thus obtained to a hydrogenolysis, to obtain the product (I).

**12.** A method according to Claim 8, characterized in that the transformation of the compounds of formula (IV) into the compounds of formula (I) is carried out by firstly reacting the compound (IV) with cyanotrimethylsilane in the presence of a catalytic amount of zinc iodide, then by subjecting the product of this reaction to a hydrolysis in an aqueous acidic medium, in such a manner as to achieve the deacetalisation of the group B, to form eventually an intermediate compound of the cyanohydrine type of the formula

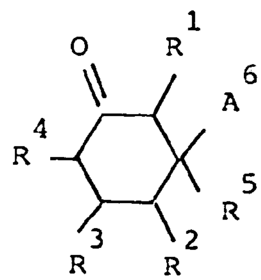

(XVI)

in which $A^6$ represents a group

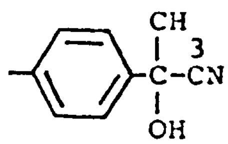

and $R^1$, $R^2$, $R^3$, $R^4$ and $R^5$ are such as defined in Claim 1, and by subjecting the intermediate compound (XVI) thus obtained to a hydrolysis and a hydrogenolysis, to form the final compound (I).

**13.** Compounds of the formula

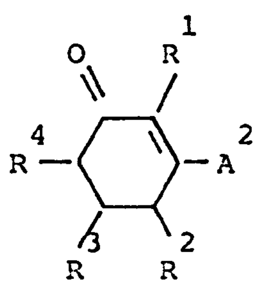

(II)

in which $A^2$ represents the group

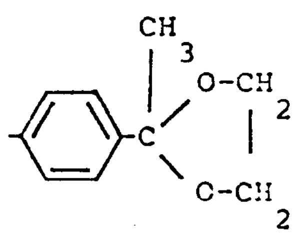

and $R^1$, $R^2$, $R^3$, $R^4$, which can be identical or different, represent each a hydrogen or an alkyl group.

**14.** Compounds of the formula

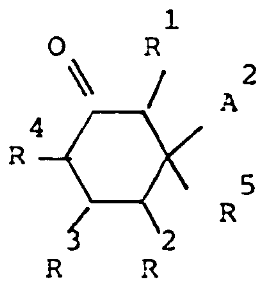

(III)

in which $A^2$ represents the group

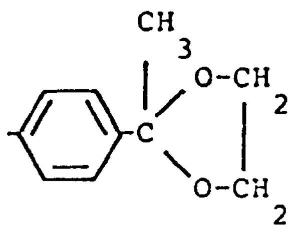

and $R^1$, $R^2$, $R^3$, $R^4$ and $R^5$, which can be identical or different, represent each a hydrogen or an alkyl group.

**15.** Compounds of the formula

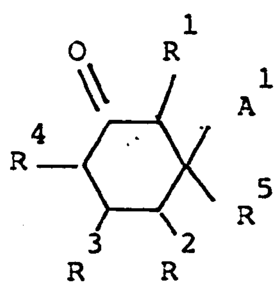

(VII)

in which $A^1$, $R^1$, $R^2$, $R^3$, $R^4$ and $R^5$ are such as defined in Claim 1.

**16.** Compounds of the formula

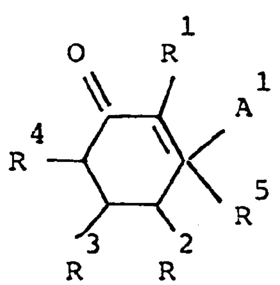

(VIII)

in which $A^1$, $R^1$, $R^2$, $R^3$ and $R^4$ are such as defined in Claim 1.

**17.** Compounds of formula

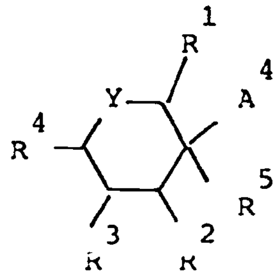

(IX), (X), (XI)
(XII), (XIII), (XIV)

in which Y represents either the group B defined in Claim 1, or a group >C=O and $A^3$ represents a group

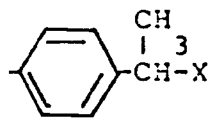

in which X represents OH, Cl or CN, the meaning of Y and X for each for formula being that indicated in the following table :

| Y = | | B | 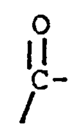 |
|---|---|---|---|
| X = | OH | (IX) | (XII) |
| | Cl | (X) | (XIII) |
| | CN | (XI) | (XIV) |

## Patentansprüche

1. Verbindungen der Formel

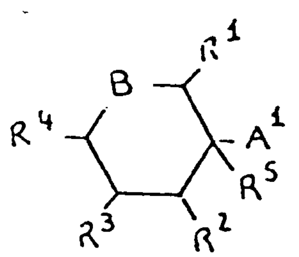

(IV)

in der $A^1$ eine Gruppe

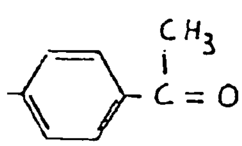

und B eine Gruppe

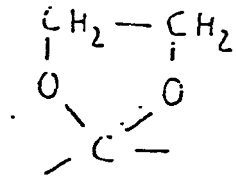

darstellt und in der $R^1$, $R^2$, $R^3$, $R^4$ und $R^5$, welche gleich oder unterschiedlich sein können, jeweils Wasserstoff oder eine Alkyl-Gruppe darstellen.

2. Verfahren zur Herstellung der Verbindungen der Forml (IV) nach Anspruch 1, dadurch gekennzeichnet, daß

(a) man ein Organomagnesium-Derivat des p-Brom-acetophenondioxolan der Formel

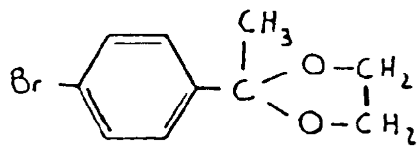

(V)

herstellt und man dieses Organomagnesium-Derivat umsetzt mit einer Verbindung der Formel

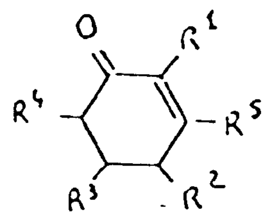

(VIa)

in der $R^1$, $R^2$, $R^3$, $R^4$ und $R^5$ die gleiche Bedeutung wie im Anspruch 1 haben, derart, daß man eine Verbindung der Formel

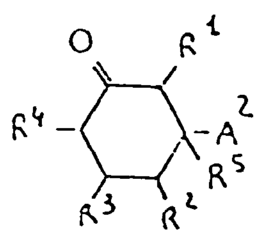

(III)

erhält, in der $A^2$ die Gruppe

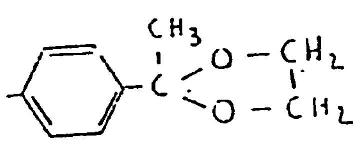

darstellt und

(b) die Verbindung der Formel (III) in eine Verbindung der Formel (IV) umwandelt.

3. Verfahren zur Herstellung der Verbindungen der Formel (IV) nach dem Anspruch 1, in der $R^5$ Wasserstoff darstellt, dadurch gekennzeichnet, daß

(a) man ein Organomagnesium-Derivat des p-Bromacetophenondioxolan der Formel

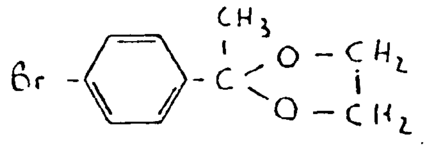

(V)

herstellt und man dieses Organomagnesium-Derivat umsetzt mit einer Verbindung der Formel (VI),

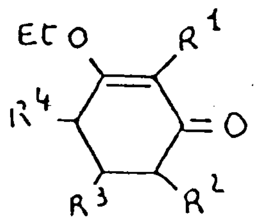

(VI)

worin Et eine Ethyl-Gruppe darstellt und $R^1$, $R^2$, $R^3$, $R^4$ die gleiche Bedeutung wie im Anspruch 1 haben, derart, daß man eine Verbindung der Formel

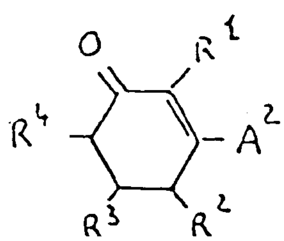

(II)

in der $A^2$ die Gruppe

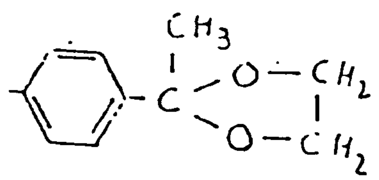

darstellt,
(b) man die Verbindung der Formel (II) reduziert, um eine Verbindung der Formel

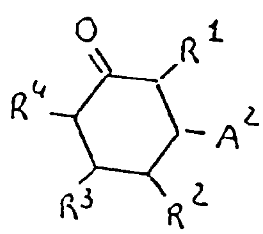

(IIIa)

zu erhalten und
(c) man die Verbindung der Formel (IIIa) in eine Verbindung der Formel (IV), in welcher $R^5$ Wasserstoff ist, umwandelt.

**4.** Verfahren nach Anspruch 2 oder Anspruch 3, dadurch gekennzeichnet, daß man die Umwandlung der Verbindung der Formel (III) oder (IIIa) in die Verbindung der Formel (IV) in einem einzigen Transacetalisierungs-Vorgang durchführt, der durch die Gegenwart einer Lewis-Säure und von Ethylenglycol katalysiert ist.

5. Verfahren nach Anspruch 2 oder Anspruch 3, dadurch gekennzeichnet, daß man die Umwandlung der Verbindung der Formel (III) oder (IIIa) in die Verbindung der Formel (IV) durchführt, indem man zuerst durch Deacetalisierung der Gruppe $A^2$ die Verbindung der Formel (III) oder (IIIa) in die Verbindung the Formel

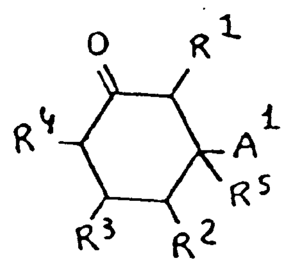

(VII)

umwandelt, dann die letztere Verbindung in eine Verbindung der Formel (IV) durch selektive Acetalisierung der Keton-Funktion der Cyclohexyl-Gruppe umwandelt.

6. Verfahren zur Herstellung der Verbindungen der Formel (IV) nach Anspruch 1, in der $R^5$ Wasserstoff darstellt, dadurch gekennzeichnet, daß
(a) man ein Organomagnesium-Derivat von p-Bromacetophenondioxolan der Formel

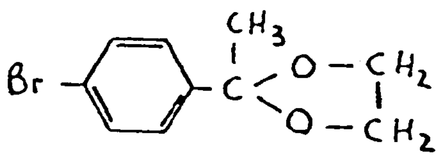

(V)

herstellt und man dieses Organomagnesium-Derivat mit einer Verbindung der Formel

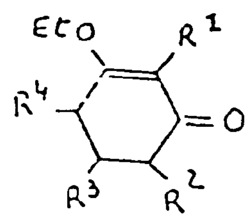

(VI)

umsetzt, in welcher Et eine Ethyl-Gruppe darstellt und $R^1$, $R^2$, $R^3$, $R^4$ die gleiche Bedeutung wie im Anspruch 1 haben, um eine Verbindung der Formel

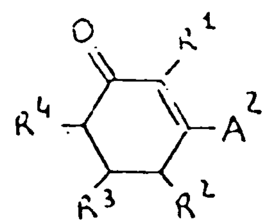

(II)

zu erhalten, in der $A^2$ die Gruppe

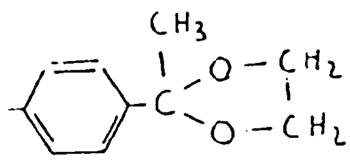

darstellt,

(b) durch Deacetalisierung der Gruppe $A^2$ die Verbindung der Formel (II) in eine Verbindung der Formel

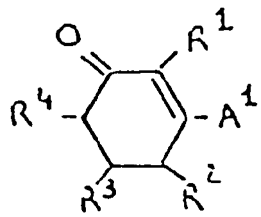

(VIII)

umwandelt,

(c) die Verbindung der Formel (VIII) reduziert, um eine Verbindung der Formel

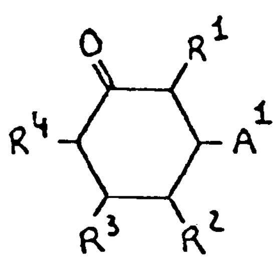

(VIIa)

zu erhalten, und

(d) letzere Verbindung in eine Verbindung der Formel (IV), in der $R^5$ Wasserstoff darstellt, durch Acetalisierung der Keton-Funktion der Cyclohexyl-Gruppe umwandelt.

7. Verfahren zur Herstellung der Verbindungen der Formel (IV) nach Anspruch 1, dadurch gekennzeichnet, daß man durch eine Friedel-Crafts-Reaktion eine Verbindung der Formel

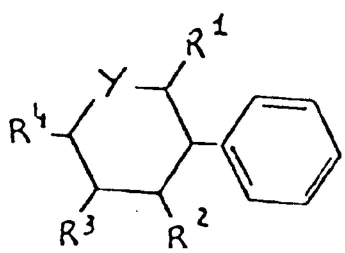

(XVII)

in der Y entweder die Gruppe B, die im Anspruch 1 definiert ist, oder eine Gruppe >C = O ist, mit Aluminiumchlorid und einer Verbindung, welche ausgewählt wird aus Acetylchlorid und Essigsäureanhydrid, umsetzt und dann das Produkt dieser Reaktion einer Hydrolyse unterwirft, gefolgt von einer Acetalisierung, um das Endprodukt der Formel (IV) zu bilden.

8. Verfahren zur Herstellung der [(Oxo-3-cyclohexyl)-4-phenyl]-2-propionsäure und deren Derivate der Formel

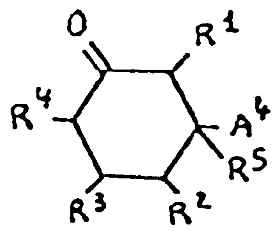

(I) .

in der $A^4$ eine Gruppe

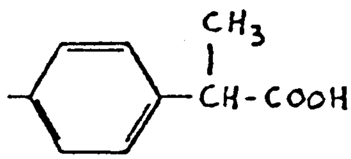

darstellt und $R^1$, $R^2$, $R^3$, $R^4$, $R^5$, welche gleich oder unterschiedlich sein können, jeweils Wasserstoff oder eine Alkyl-Gruppe darstellen, dadurch gekennzeichnet, daß man die Verbindungen der Formel (IV), welche im Anspruch 1 definiert sind, in die Verbindungen der Formel (I) umsetzt.

**9.** Verfahren nach Anspruch 8, dadurch gekennzeichnet, daß man die Umsetzung der Verbindungen der Formel (IV) in Verbindungen der Formel (I) durchführt, indem man nacheinander Zwischenverbindungen der Formel

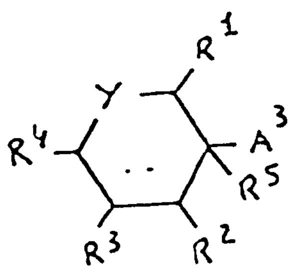

(IX), (X), (XI) (XII), (XIII), (XIV)

bildet, in der Y entweder die im Anspruch 1 definierte Gruppe B oder eine Gruppe

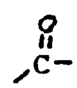

darstellt und $A^3$ eine Gruppe

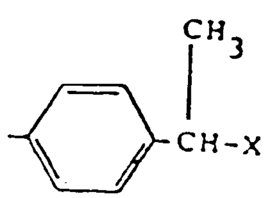

darstellt, in der X OH, Cl oder CN darstellt, wobei die Bedeutungen von Y und X für jede Formel diejenigen sind, welche in der folgenden Tabelle angegeben sind:

| Y = | B | 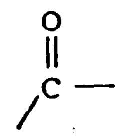 |
|---|---|---|
| X = OH | (IX) | (XII) |
| Cl | (X) | (XIII) |
| CN | (XI) | (XIV) |

und zwar eine erste Zwischenverbindung (IX) oder (XII), in der X OH darstellt, erhalten durch Reduktion der Keton-Funktion der Gruppe $A^1$, und im Fall der Deacetalisierung der Acetal-Funktion der Gruppe B eine zweite Zwischenverbindung (X) oder (XIII), in der X Cl darstellt, erhalten durch Substitution der OH-Gruppe der ersten Zwischenverbindung durch Chlor, und eine dritte Zwischenverbindung (XI) oder (XIV), in der X CN darstellt, erhalten durch Substitution des Chlors der zweiten Zwischenverbindung durch die CN-Gruppe, worauf die dritte Zwischenverbindung umgewandelt wird in eine Verbindung der Formel (I), entweder direkt in dem Fall, wo Y die Gruppe

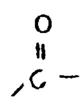

ist, oder aber, in dem Fall, wo Y die Gruppe B darstellt, durch Deacetalisierung der letzteren Gruppe.

**10.** Verfahren nach Anspruch 8, dadurch gekennzeichnet, daß man eine Umwandlung der Verbindungen der Formel (IV) in Verbindungen der Formel (I) durchführt, indem nacheinander die Zwischenverbindungen der Formel

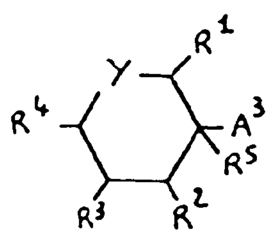

(IX), (X), (XII), (XIII)

gebildet werden, in denen Y entweder die im Anspruch 1 definierte Gruppe B oder eine Gruppe

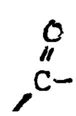

darstellt, und $A^3$ eine Gruppe

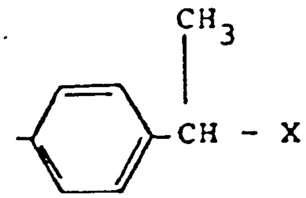

darstellt, in der X OH oder Cl darstellt, wobei die Bedeutungen von Y und X für jede Formel diejenigen sind, die in der nachstehenden Tabelle angegeben sind:

| | | | |
|---|---|---|---|
| Y = | | B | 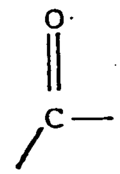 |
| X = | OH | (IX) | (XII) |
| | Cl | (X) | (XIII) |

und zwar eine erste Zwischenverbindung (IX) oder (XII), in der X OH darstellt, erhalten durch Reduktion der Keton-Funktion der Gruppe $A^1$ und eine zweite Zwischenverbindung (X) oder (XIII), in der X Cl darstellt, erhalten durch Substitution der OH-Gruppe der ersten Zwischenverbindung durch Chlor, indem man anschließend ggf. die Verbindung (XIII) durch Acetalisierung der Keton-Funktion Y in die Verbindung (X) umwandelt, worauf man die Verbindung (X) in eine Verbindung der Formel (I) durch eine Grignard-Reaktion umwandelt, nach der man das Organomagnesium-Derivat der Verbindung (X) mit Kohlensäureanhydrid umsetzt und man das so erhaltene Additionsprodukt hydrolisiert, indem man u. a. eine Deacetalisierung der Gruppe B in der Weise durchführt, um das Endprodukt (I) zu erhalten.

**11.** Verfahren nach Anspruch 8, dadurch gekennzeichnet, daß man die Umwandlungen der Verbindung der Formel (IV) in eine Zwischenverbindung der Formel

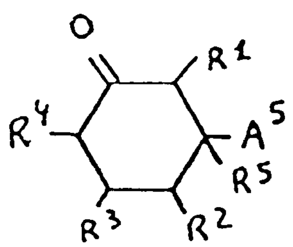

(XV)

durchführt, in der $A^5$ eine Gruppe

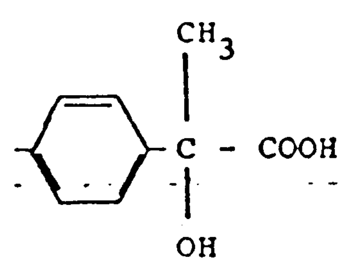

darstellt und $R^1$, $R^2$, $R^3$, $R^4$, $R^5$ so definiert sind wie im Anspruch 1, wobei die Zwischenverbindung durch Reaktion der Verbindung (IV) mit Chloroform und einem Alkalimetallhydroxyd in Gegenwart einer katalytischen Menge Benzyltriethylaminchlorid erhalten wird, gefolgt von der Deacetalisierung der Gruppe B der Verbindung (IV) durch Hydrolyse in einem sauren wäßrigen Milieu, und indem man die so erhaltene Zwischenverbindung (XV) einer Hydrogenolyse unterwirft, um das Produkt (I) zu bilden.

**12.** Verfahren nach Anspruch 8, dadurch gekennzeichnet, daß man eine Umwandlung der Verbindungen der Formel (IV) in Verbindungen der Formel (I) durchführt, indem man zuerst die Verbindung (IV) mit Cyanotrimethylsilan in Gegenwart einer katalytischen Menge Zinkjodid umsetzt, indem man dann das Produkt dieser Reaktion einer Hydrolyse in einem sauren wäßrigen Milieu unterwirft, um eine Deacetalisierung der Gruppe B durchzuführen, um eine Zwischenverbindung vom Cyanohydrin-Typ der Formel

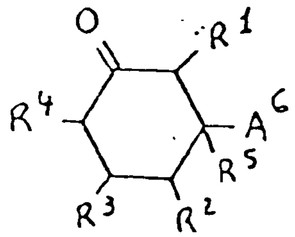

(XVI)

zu bilden, in der $A^6$ eine Gruppe

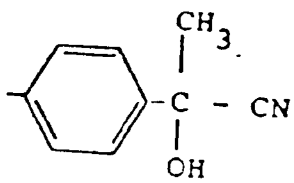

darstellt und $R^1$, $R^2$, $R^3$, $R^4$ und $R^5$ so definiert sind wie im Anspruch 1, und indem man die so erhaltene Zwischenverbindung (XVI) einer Hydrolyse oder einer Hydrogenolyse unterwirft, um das Endprodukt (I) zu bilden.

**13.** Verbindungen der Formel

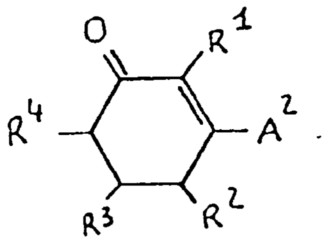

(II)

in der $A^2$ die Gruppe

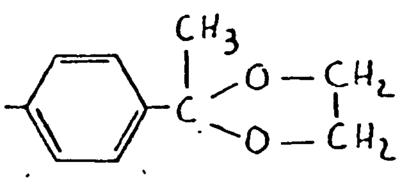

darstellt und $R^1$, $R^2$, $R^3$, $R^4$, welche gleich oder unterschiedlich sein können, jeweils Wasserstoff oder eine Alkyl-Gruppe darstellen.

**14.** Verbindungen der Formel (III)

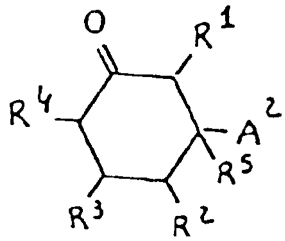

(III)

in der $A^2$ die Gruppe

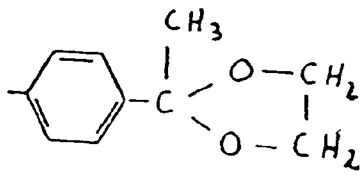

darstellt und $R^1$, $R^2$, $R^3$, $R^4$ und $R^5$, welche gleich oder unterschiedlich sein können, jeweils Wasserstoff oder eine Alkyl-Gruppe darstellen.

**15.** Verbindungen der Formel

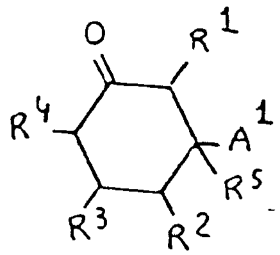

(VII)

in der $A^1$, $R^1$, $R^2$, $R^3$, $R^4$ und $R^5$ so definiert sind wie im Anspruch 1.

**16.** Verbindungen der Formel

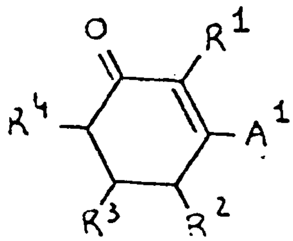

(VIII)

in der $A^1$, $R^1$, $R^2$, $R^3$ und $R^4$ so definiert sind wie im Anspruch 1.

**17.** Verbindungen der Formel

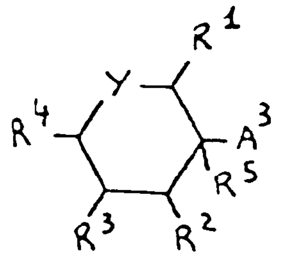

(IX), (X),
(XI), (XII),
(XIII), (XIV)

in denen Y entweder die im Anspruch 1 definierte Gruppe B oder eine Gruppe $>$C = O bedeutet, und $A^3$ eine Gruppe

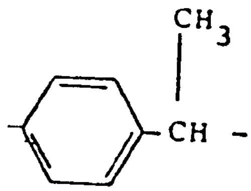

darstellt, in der X OH, Cl oder CN darstellt, wobei die Bedeutungen von Y und X für jede Formel diejenigen sind, welche in der nachstehenden Tabelle angegeben sind:

| Y = | | B | 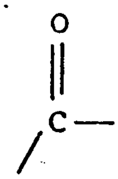 |
|---|---|---|---|
| | OH | (IX) | (XII) |
| X = | Cl | (X) | (XIII) |
| | CN | (XI) | (XIV) |

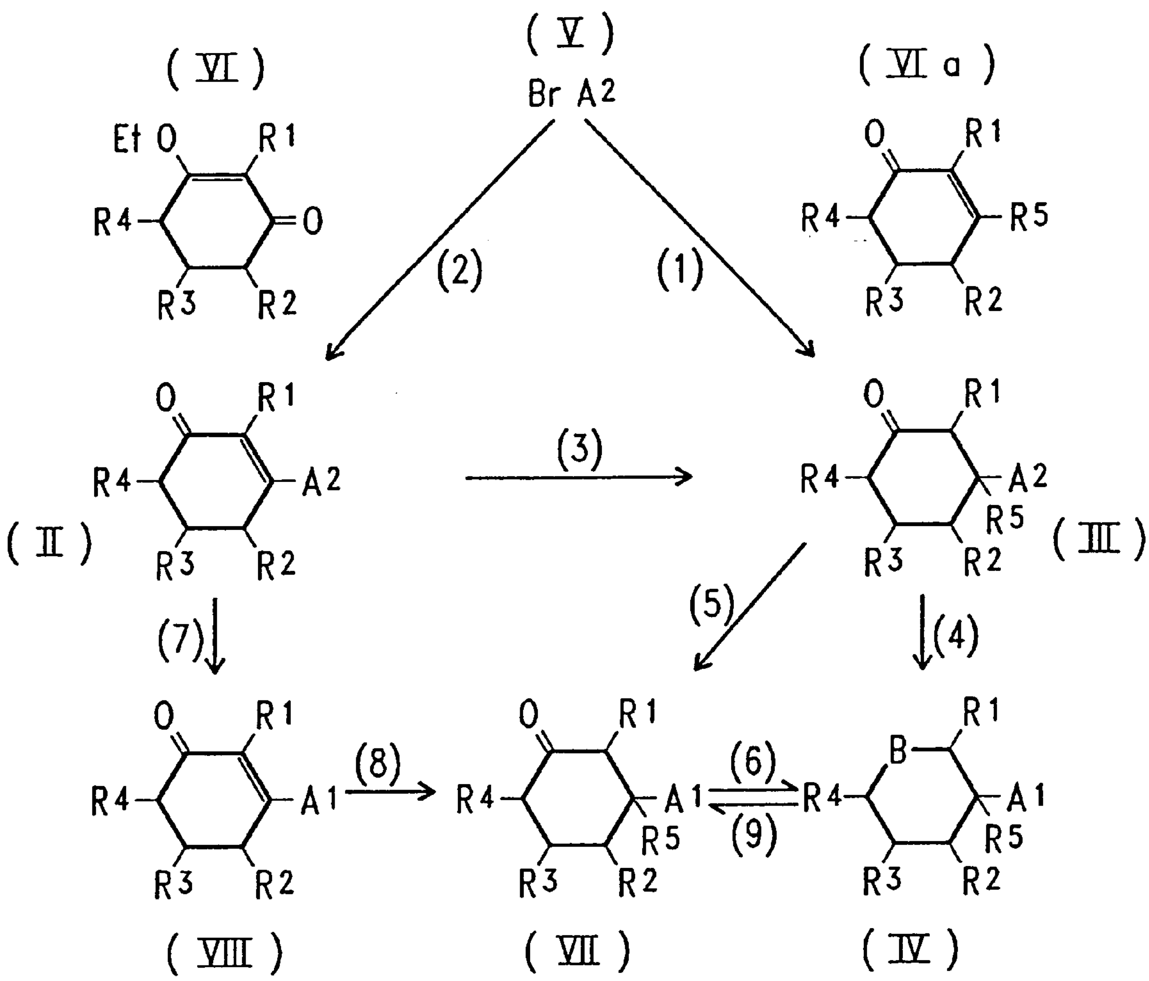


Fig. 1

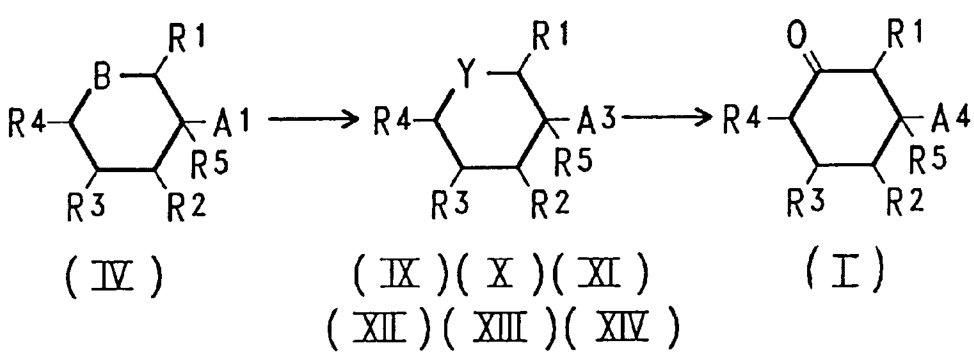


Fig. 2

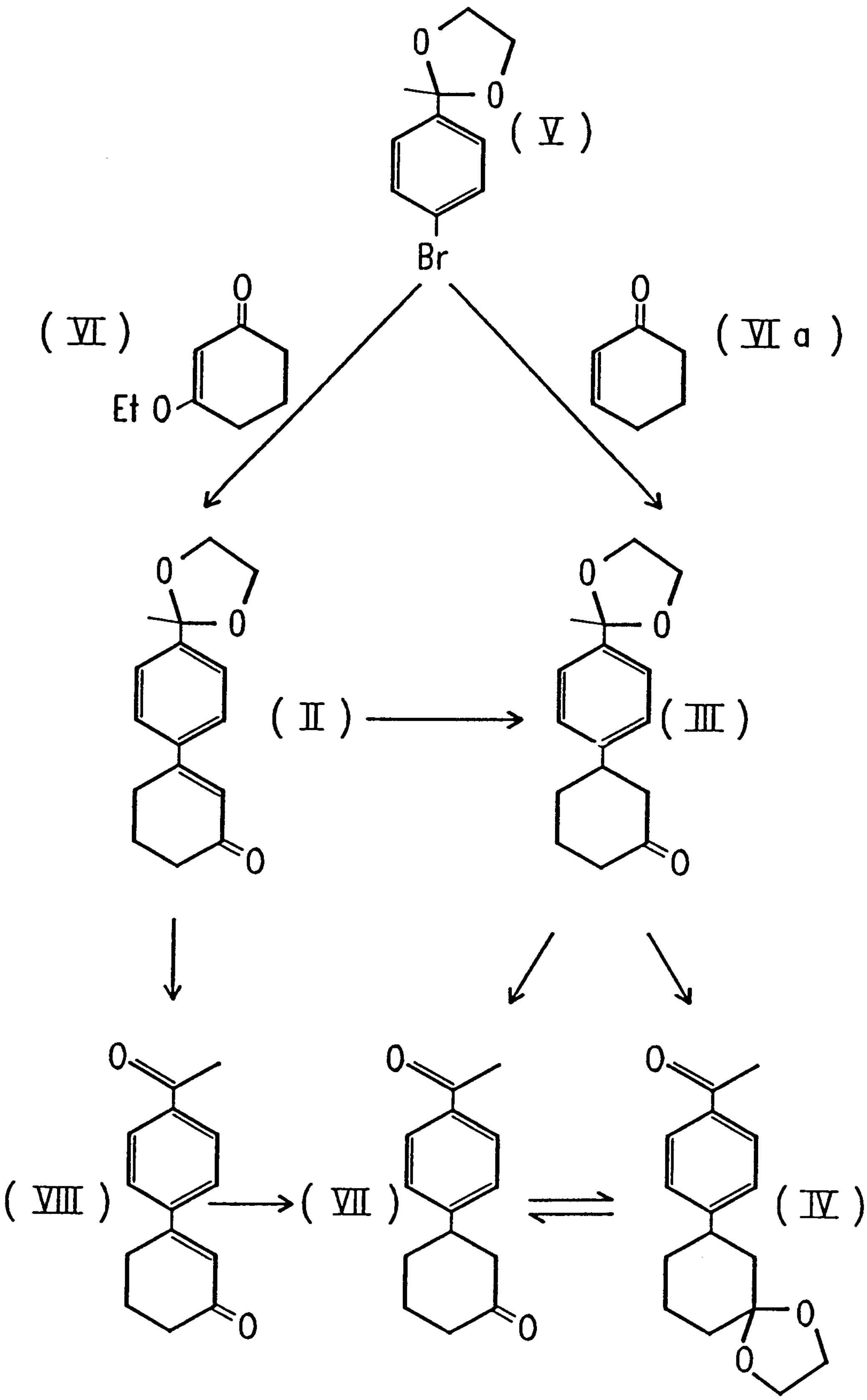

(V)

(VI) (VIa)

(II) (III)

(VIII) (VII) (IV)

Fig. 3

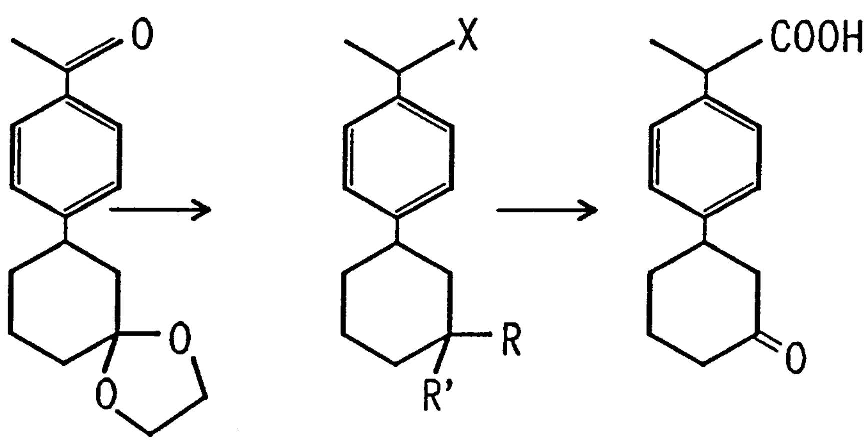

(IV) (I)

RR'= DIOXOLAN ou =O

(IX) X = OH (XII)

(X) X = Cl (XIII)

(XI) X = CN (XIV)

Fig. 4